# FASCICULE DE BREVET EUROPEEN

(11) **EP 1 322 673 B1**
(45) Date de publication et mention de la délivrance du brevet: **26.09.2007**
(21) Numéro de dépôt: 01972171.1
(22) Date de dépôt: 20.09.2001
(51) Int. Cl.: C08B 37/00, A61K 31/715

(54) **POLYSACCHARIDES A ACTIVITE ANTITHROMBOTIQUE COMPRENANT AU MOINS UNE LIAISON COVALENTE AVEC LA BIOTINE OU UN DERIVE DE LA BIOTINE**
POLYSACCHARIDE MIT ANTITHROMBOTISCHER WIRKUNG, DIE MIT BIOTIN ODER EINEM BIOTENDERIVAT KOVALENT GEBUNDEN SIND
POLYSACCHARIDES WITH ANTITHROMBOTIC ACTIVITY COMPRISING AT LEAST A COVALENT BOND WITH BIOTIN OR A BIOTIN DERIVATIVE

(30) Priorité: 22.09.2000 FR 0012094
(43) Date de publication de la demande: 02.07.2003
(73) Titulaire: Sanofi-Aventis, 75013 Paris (FR)
(72) Inventeur: DUCHAUSSOY, Philippe, F-31200 Toulouse (FR); HERBERT, Jean-Marc, F-31170 Tournefeuille (FR); PETITOU, Maurice, F-75645 Paris Cedex 13 (FR); SAVI, Pierre, F-31600 Seysses (FR)
(74) Mandataire: Tsitini-Souleau, Maria
(86) Numéro de dépôt international: PCT/FR2001/002918
(87) Numéro de publication internationale: WO 2002/024754

(56) Documents cités:
- WO-A-99/36443

## Description

La présente invention concerne des nouveaux oligo - et polysaccharides de synthèse présentant au moins une liaison covalente avec la biotine ou un dérivé de la biotine et possédant les activités pharmacologiques anticoagulante et antithrombotique de l'héparine.

L'héparine catalyse, notamment *via* l'antithrombine III (AT III), l'inhibition de deux enzymes qui interviennent dans la cascade de la coagulation du sang, à savoir, le facteur Xa et le facteur IIa (ou thrombine). Les préparations d'héparines de bas poids moléculaire (HBPM), contiennent des chaînes formées de 4 à 30 monosaccharides et ont la propriété d'agir plus sélectivement sur le facteur Xa que sur la thrombine.

Il est connu que l'inhibition du facteur Xa nécessite une fixation de l'héparine sur l'AT III *via* le domaine de liaison à l'antithrombine (Domaine-A), et que l'inhibition du facteur IIa (thrombine) nécessite une fixation à l'AT III, *via* le Domaine-A, ainsi qu'à la thrombine *via* un domaine de liaison moins bien défini (Domaine-T).

Les oligosaccharides de synthèse correspondant au domaine Domaine-A de l'héparine sont connus. Ils sont décrits par exemple dans les brevets EP 84999 et EP 529715, la demande de brevet publiée sous le numéro WO 99/36428 et la publication *Bioorg.*

*Med. Chem.* 1998, 6,1509-1516. Ces oligosaccharides de synthèse ont la propriété d'inhiber sélectivement, *via* l'antithrombine III, le facteur Xa de la coagulation sans aucune activité sur la thrombine. Ils manifestent une activité antithrombotique dans la thrombose veineuse.

Des oligosaccharides de synthèse capables d'inhiber la thrombine et le facteur Xa *via* l'activation de l'AT III ont été décrits dans les demandes de brevet publiées sous les numéros WO 98/03554 et WO 99/36443.

Dans ces demandes de brevet sont décrits de nouveaux dérivés polysaccharidiques sulfatés et alkylés biologiquement actifs. Ils sont en particulier anticoagulants et antithrombotiques. Il a en particulier été montré que ces polysaccharides sulfatés et alkylés peuvent être de puissants antithrombotiques et des anticoagulants selon la disposition des groupes alkyles et des groupes sulfates portés par le squelette glucidique. De façon plus générale, il a été trouvé que par réalisation de séquences polysaccharidiques il est possible de moduler avec précision les activités de type GAGs pour obtenir des produits très actifs présentant les propriétés pharmacologiques anticoagulantes et antithrombotiques de l'héparine. Par rapport à l'héparine, ils présentent l'avantage d'être d'une structure déterminée et de ne pas réagir avec le facteur 4 plaquettaire, cause des effets thrombocytopéniants de l'héparine.

Cependant, l'usage en thérapeutique humaine de certains produits décrits dans les demandes de brevet publiées sous les numéros WO 98/03554 et WO 99/36443 et dans le brevet EP 529715 peut s'avérer délicate, en particulier si ces produits possèdent une longue demi-vie. Dans le domaine de la prévention ou du traitement de la thrombose avec les produits ci-dessus, on doit rétablir ou maintenir la fluidité du sang tout en évitant de provoquer une hémorragie.

Il est en effet bien connu que, pour une cause accidentelle quelconque, une hémorragie peut se déclencher chez un patient sous traitement. On peut également avoir besoin d'intervenir chirurgicalement chez un malade sous traitement antithrombotique. De plus, au cours de certains actes chirurgicaux, des anticoagulants peuvent être utilisés à forte dose de façon à empêcher la coagulation du sang, et il est nécessaire de les neutraliser à la fin de l'intervention. Il est donc intéressant d'avoir des agents antithrombotiques neutralisables pour stopper l'activité anticoagulante à tout moment. Or, les oligosaccharides de synthèse connus, décrits précédemment, ne peuvent pas être aisément neutralisés par les antidotes connus de l'héparine ou des HBPM, y compris le sulfate de protamine.

La présente invention concerne de nouveaux polysaccharides de synthèse, de structure proche de celle des composés décrits dans les demandes de brevet publiées sous les numéros WO 98/03554 et WO 99/36443 et dans le brevet EP 529715 : les structures des oligosaccharides de synthèse objet de l'invention sont modifiées, en ce sens qu'ils présentent une liaison covalente avec la biotine (acide hexahydro-2-oxo-*1H*-thiéno[3,4-d]imidazole-4-pentanoïque) ou avec un dérivé de la biotine. De manière surprenante, il apparaît que l'introduction de biotine ou d'un dérivé de la biotine ne modifie pas l'activité pharmacologique des polysaccharides. En effet, les nouveaux polysaccharides, objet de l'invention, ont une activité antithrombotique comparable aux oligosaccharides de l'art antérieur. Mais ils possèdent en outre l'avantage de pouvoir être rapidement neutralisés par un antidote spécifique, en situation d'urgence. Cette antidote spécifique est l'avidine (The Merck Index, Twelfth edition, 1996, M.N. 920, pages 151-152) ou la streptavidine, deux protéines tétramériques de masses respectives égale à environ 66 000 et 60 000 Da, qui possèdent une très forte affinité pour la biotine.

D'une manière générale, l'invention concerne des polysaccharides synthétiques à activité antithrombotique présentant au moins une liaison covalente avec la biotine ou un dérivé de la biotine.

A titre de dérivé de la biotine, on peut citer les dérivés de la biotine indiqués dans le catalogue Pierce 1999-2000 pages 62 à 81, par exemple le 6-biotinamido hexanoate, 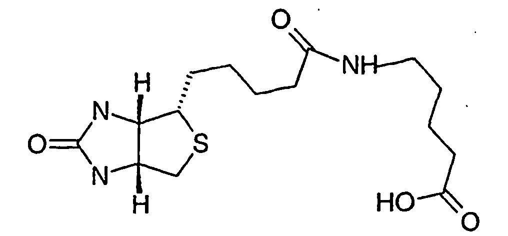 ou le 6-(6-biotinamidohexanamido) hexanoate, 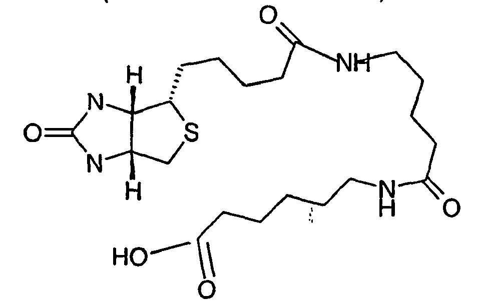 ou encore le 2-biotinamido éthanethiole 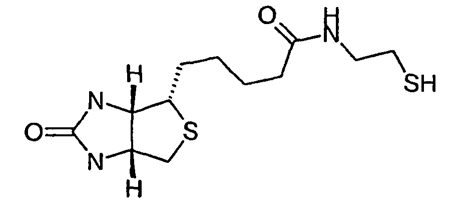 ou encore les composés de formules suivantes :

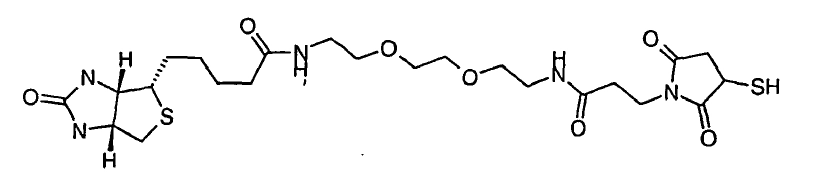

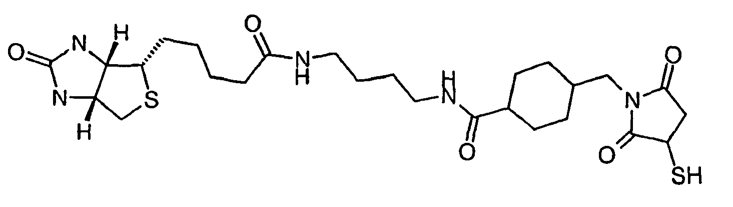

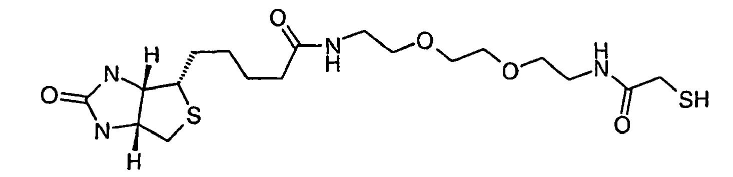

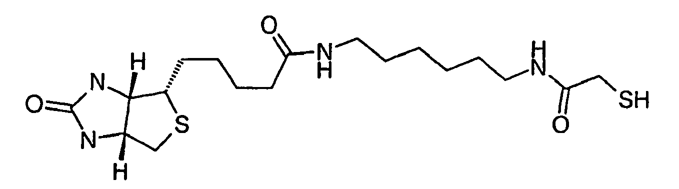

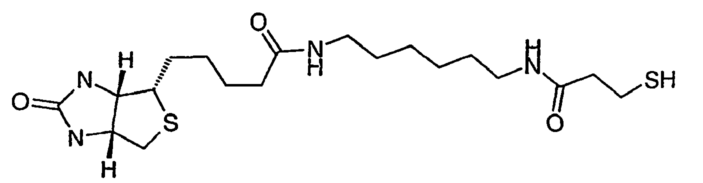

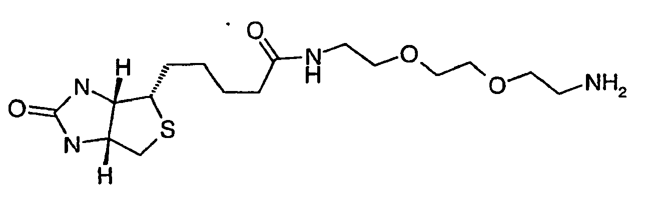

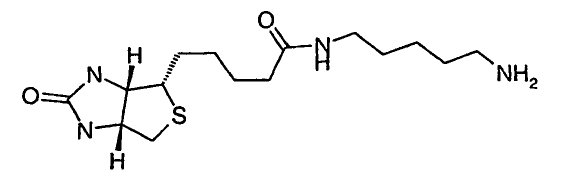

En particulier, la présente invention a pour objet les polysaccharides de formule (I) : 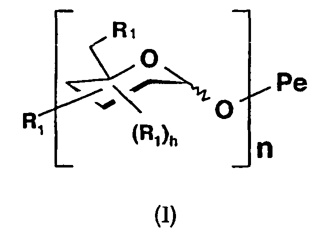 dans laquelle :
- le trait ondulé désigne une liaison située soit au-dessous soit au-dessus du plan du cycle pyranosique, 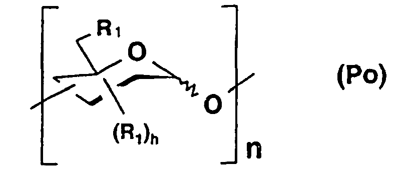
   Po désigne un polysaccharide, contenant n unités monosaccharidiques identiques ou différentes, lié par son carbone anomère à Pe, 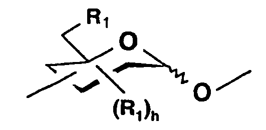 est une représentation schématique d'une unité monosaccharidique à structure pyranosique choisie parmi les hexoses, les pentoses et les sucres desoxy correspondants, cette unité étant liée par son carbone anomère à une autre unité monosaccharidique, et les groupes hydroxy de cette unité étant substitués par des groupes R₁ identiques ou différents, R₁ étant tel que défini ci-dessous,
- Pe représente un pentasaccharide de structure : 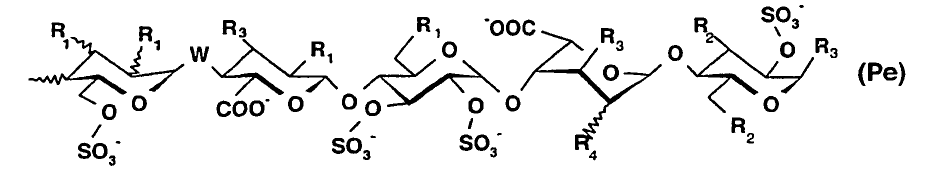
- h est égal à 1 ou 2,
- n est un entier et peut prendre toute valeur de 0 à 25,
- R₁ représente l'enchaînement -T-Biot, un groupe (C₁-C₆)alcoxy ou un groupe -OSO₃⁻,
- R₂ représente l'enchaînement -T-Biot, un groupe (C₁-C₆)alcoxy ou un groupe -OSO₃⁻,
- R₃ représente l'enchainement -T-Biot, un groupe (C₁-C₆)alcoxy,
- R₄ représente l'enchaînement -T-Biot, un groupe (C₁-C₆)alcoxy ou un groupe -OSO₃⁻ ou bien R₄ constitue un pont -O-CH₂-, le groupe -CH₂- étant lié à l'atome de carbone porteur de la fonction carboxylique sur le même cycle ; étant entendu que l'un au moins des substituants R₁, R₂, R₃ ou R₄ représente un groupe -T-Biot,
- W représente un atome d'oxygène ou un groupe méthylène,
- T représente un des enchaînements choisis parmi: NH, 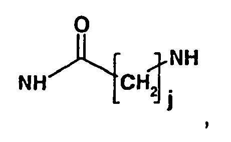 ou 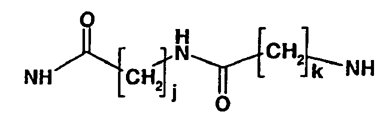 dans lesquels j et k, identiques ou différents, sont des entiers pouvant prendre toute valeur de 1 à 10 ;
- Biot représente le groupe : 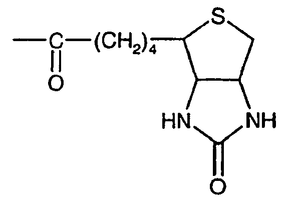
ainsi que leurs sels pharmaceutiquement acceptables.

Comme indiqué précédemment, on notera que de façon générale dans la présente description un trait ondulé désigne une liaison située, soit au-dessous, soit au-dessus du plan du cycle pyranosique.

Les monosaccharides contenus dans Po peuvent être identiques ou différents les uns des autres, les liaisons interglycosidiques peuvent être du type α ou β.

Ces monosaccharides sont avantageusement choisis parmi les hexoses D ou L alose, altrose, glucose, mannose, galose, idose, galactose, talose (dans ce cas h = 2) ou parmi les pentoses D ou L ribose, arabinose, xylose, lyxose (dans ce cas h = 2). D'autres monosaccharides tels que par exemple les sucres désoxy peuvent également être utilisés (h = 1 et/ou -CH₂R₁ = CH₃).

La partie polysaccharidique Po peut être constituée de 0 à 25 unités monosaccharidiques alkylées et di- ou trisulfatées.

La partie polysaccharidique Po peut être également constituée de 0 à 25 unités monosaccharidiques alkylées et mono- ou disulfatées.

La partie polysaccharidique Po peut être constituée de 0 à 25 unités monosaccharidiques alkylées non chargées et/ou partiellement chargées et/ou totalement chargées.

Les unités chargées ou non chargées peuvent être dispersées tout au long de la chaîne ou elles peuvent au contraire être groupées en domaines saccharidiques chargés ou non chargés.

Les liaisons entre les unités peuvent être 1,2 ; 1,3 ; 1,4 ; 1,5 ; 1,6 ; et du type α ou β.

Dans la présente description, il a été choisi de représenter les conformations ¹C₄ pour l'acide-L-iduronique, ⁴C₁ pour l'acide D-glucuronique, mais il est notoire que, d'une façon générale, la conformation en solution des unités monosaccharides est fluctuante.

Ainsi, l'acide L-iduronique peut être de conformation ¹*C*₄ ²*S*₀ ou ⁴*C*₁.

Selon un de ses aspects, l'invention concerne les polysaccharides de formule (I.1) : 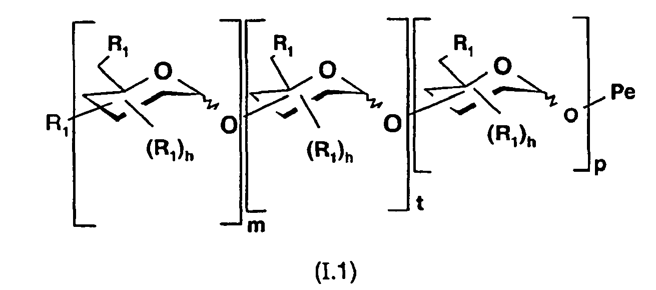 dans lesquels: 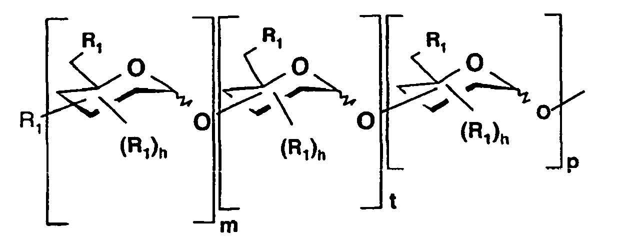 désigne une famille particulière de polysaccharides Po, liés par leur carbone anomère à Pe tel que défini pour (I), 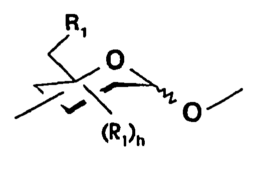 est tel que défini pour (I),
- les groupes R₁ sont tels que définis pour (I) et, pour un même monosaccharide, peuvent être identiques ou différents,
- le monosaccharide contenu dans [ ]ₘ est répété m fois, le monosaccharide contenu dans [ ]ₜ est répété t fois, le monosaccharide contenu dans [ ]ₚ est répété p fois,
- m est un entier variant de 1 à 5, t est un entier variant de 0 à 24 et p est un entier variant de 0 à 24 étant entendu que 1 ≤ m + t + p ≤ 25,
ainsi que leurs sels pharmaceutiquement acceptables.

Parmi ces polysaccharides de formule (I.1), les polysaccharides dans lesquels un seul des substituants R₁, R₂, R₃ ou R₄ représente l'enchaînement T-Biot avec T et Biot étant tels que définis pour (I), ainsi que leurs sels pharmaceutiquement acceptables, constituent un autre aspect de l'invention.

Selon un aspect particulier, l'invention concerne les hexadécasaccharides de formule (1.2) : 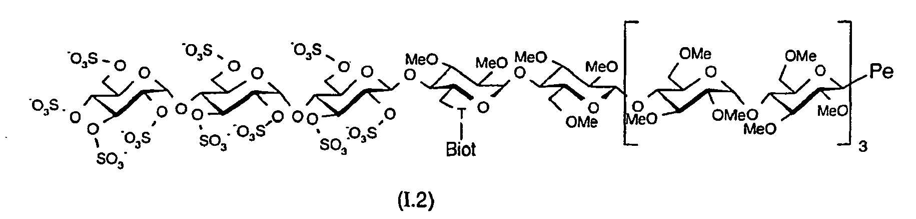 dans laquelle :
- T représente un des enchaînements choisis parmi : NH, 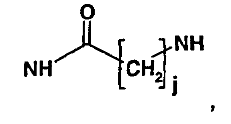
ou 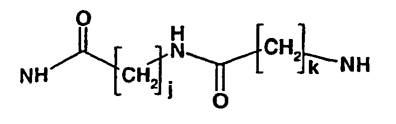 dans lesquels j et k, identiques ou différents, sont des entiers pouvant prendre toute valeur de 1 à 10 ;
- Biot représente le groupe : 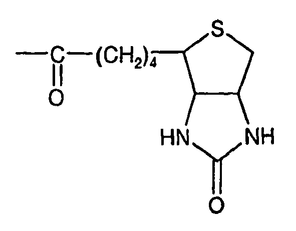
- Pe représente un pentasaccharide de structure : 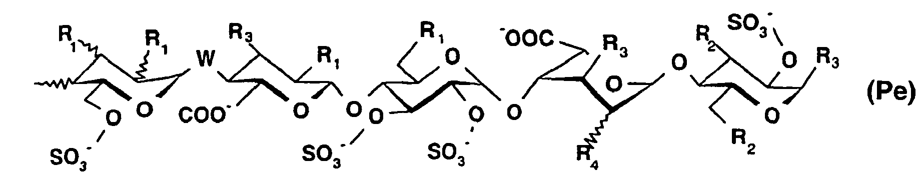
dans lequel :
- R₁ représente un groupe (C₁-C₆)alcoxy ou un groupe -OSO₃⁻,
- R₂ représente un groupe (C₁-C₆)alcoxy ou un groupe -OSO₃⁻,
- R₃ représente un groupe (C₁-C₆)alcoxy,
- R₄ représente un groupe (C₁-C₆)alcoxy ou un groupe -OSO₃⁻, ou bien R₄ constitue un pont -O-CH₂-, le groupe -CH₂- étant lié à l'atome de carbone porteur de la fonction carboxylique sur le même cycle,
- W représente un atome d'oxygène ou un groupe méthylène,
ainsi que leurs sels pharmaceutiquement acceptables.

Selon un autre de ses aspects, l'invention concerne les pentasaccharides de formule (I.3) : 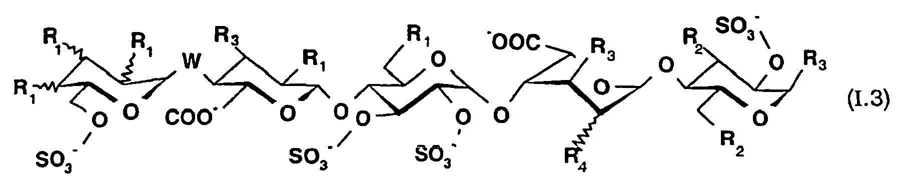 dans lesquels R₁, R₂, R₃, R₄ et W sont tels que définis pour (I), ainsi que leurs sels pharmaceutiquement acceptables.

Parmi ces pentasaccharides de formule (1.3), les pentasaccharides dans lesquels un seul des substituants R₁, R₂, R₃ ou R₄ représente l'enchaînement -T-Biot avec T et Biot étant tels que définis pour (I), ainsi que leurs sels pharmaceutiquement acceptables, constituent un autre aspect de l'invention.

Parmi ces pentasaccharides de formule (1.3), l'invention a également pour objet les pentasaccharides de formule (1.4) : 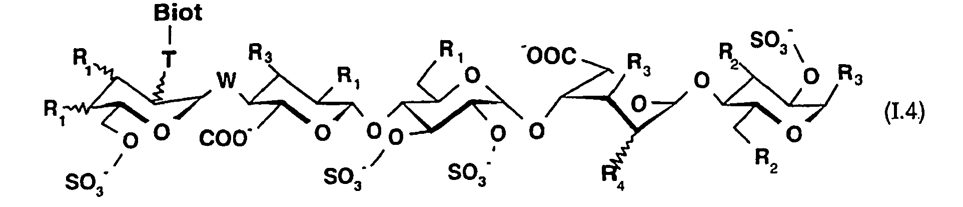 dans laquelle:
- T représente un des enchaînements choisis parmi : NH, 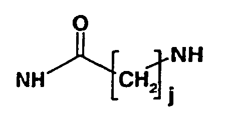
ou 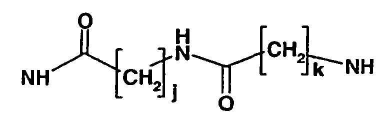 dans lesquels j et k, identiques ou différents, sont des entiers pouvant prendre toute valeur de 1 à 10 ;
- Biot représente le groupe : 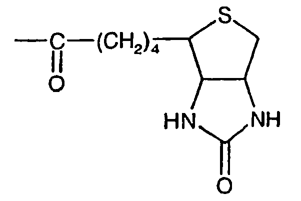
- R₁ représente un groupe (C₁-C₆)alcoxy ou un groupe -OSO₃⁻,
- R₂ représente un groupe (C₁-C₆)alcoxy ou un groupe -OSO₃⁻,
- R₃ représente un groupe (C₁-C₆)alcoxy,
- R₄ représente un groupe (C₁-C₆)alcoxy ou un groupe -OSO₃⁻, ou bien R₄ constitue un pont -O-CH₂-, le groupe -CH₂- étant lié à l'atome de carbone porteur de la fonction carboxylique sur le même cycle,
- W représente un atome d'oxygène ou un groupe méthylène, ainsi que leurs sels pharmaceutiquement acceptables.

Selon un autre de ses aspects, l'invention concerne les polysaccharides suivants :
- Méthyl (2,3,4,6-tétra-*O*-suffonato-α-D-glucopyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(6-biotinamido-6-désoxy-2,3-di-*O*-méthyl-α-D-glucopyranosyl)-(1→4)-(2,3,6-tri-*O*-méthyl-β-D-glucopyranosyl)-(1→4)-[(2,3,6-tri-*O*-méthyl-α-D-glucopyranosyl)-(1→4)-*O*-(2,3,6-tri-*O-*méthyl-β-D-glucopyranosyl)-(1→4)]₃-(6-*O*-sulfonato-2,3-di-*O*-méthyl-α-D-glucopyranosyl)-(1→4)-(acide 2,3-di-*O*-méthyl-β-D-glucopyranosyluronique)-(1→4)-(2,3,6-tri-*O-*sulfonato-α-D-glucopyranosyl)-(1→4)-(acide 2,3-di-*O*-méthyl-α-L-idopyranosyluronique)-(1→4)-2,3,6-tri-*O*-sulfonato-α-D-glucopyranoside, sel de sodium,
- Méthyl (2,3,4,6-tétra-*O*-sulfonato-α-D-glucopyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(6-[6-(biotinamidohexamido)hexamido]-6-désoxy-2,3-di-*O*-méthyl-α-D-glucopyranosyl)-(1→4)-(2,3,6-tri-*O*-méthyl-β-D-glucopyranosyl)-(1→4)-[(2,3,6-tri-*O*-méthyl-α-D-glucopyranosyl)-(1→4)-*O*-(2,3,6-tri-*O*-méthyl-β-D-glucopyranosyl)-(1→4)]₃-(6-*O-*sulfonato-2,3-di-*O*-méthyl-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O*-méthyl-α-L-idopyranosyluronique)-(1→4)-2,3,6-tri-*O*-sulfonato-α-D-glucopyranoside, sel de sodium,
- Méthyl (2,3,4,6-tétra-*O*-sulfonato-α-D-glucopyranosyl)-(1→4)-(2,3,6-tri-O-sulfonato-α-D-glucopyranosyl)-(1→4)-(2,3,6-tri-*O*-sulfonato-β-D-glucopyranosyl)-(1→4)-(6-[6-(6-biotanamidohexamido) hexamido]-6-désoxy-2,3-di-*O*-méthyl-α-D-glucopyranosyl)-(1→4)-(2,3,6-tri-*O*-méthyl-β-D-glucopyranosyl)-(1→4)-[(2,3,6-tri-*O*-méthyl-α-D-glucopyranosyl)-(1→4)-*O*-(2,3,6-tri-*O*-méthyl-β-D-glucopyranosyl)-(1→4)]₃-(6-*O*-sulfonato-2,3-di-*O*-méthyl-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O-*méthyl-α-L-idopyranosyluronique)-(1→4)-2,3,6-tri-*O*-sulfonato-α-D-glucopyranoside, sel de sodium,
- Méthyl (2-biotinamido-2-désoxy-3,4-di-O-méthyl-6-*O*-sulfonato-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O*-méthyl-α-L-idopyranosyluronique)-(1→4)-2,3,6-tri-*O*-sulfonato-α-D-glucopyranoside, sel de sodium,
- Méthyl (2-[N-(6-biotinamido hexanoyl)]-2-désoxy-3,4-di-O-méthy)-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)- 2,3,6-tri-O-sulfonato-α-D-glucopyranoside, sel de sodium
- Méthyl (2-[6-(6-biotinamidohexamido) hexamido]-2-désoxy-3,4-di-O-méthyl-6-*O-*sulfonato-α-D-glucopyranosyl)-(1→4)-(acide 2,3-di-O-méthyl-β-D-glucopyranosyluronique)-(1→4)-(2,3,6-tri-*O*-sulfonato-α-D-glucopyranosyl)-(1→4)-(acide 2,3-di-*O-*méthyl-α-L-idopyranosyluronique)-(1→4)-2,3,6-tri-*O*-sulfonato-α-D-glucopyranoside, sel de sodium.

L'invention englobe les polysaccharides sous leur forme acide ou sous la forme de l'un quelconque de leurs sels pharmaceutiquement acceptables. Dans la forme acide, les fonctions -COO⁻ et -SO₃⁻ sont respectivement sous forme -COOH et -SO₃H.

On entend par sel pharmaceutiquement acceptable des polysaccharides de l'invention, un polysaccharide dans lequel une ou plusieurs des fonctions -COO- ou/et -SO₃⁻ sont liées de façon ionique à un cation pharmaceutiquement acceptable. Les sels préférés selon l'invention sont ceux dont le cation est choisi parmi les cations des métaux alcalins et plus préférablement encore ceux dont le cation est Na⁺ ou K⁺.

Les composés de la formule (I) ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, dans les essais biochimiques en tant que ligands.

Dans son principe le procédé de préparation des composés selon l'invention utilise des synthons de base di- ou oligosaccharidiques préparés comme précédemment rapporté dans la littérature. On se réfèrera notamment aux brevets ou demandes de brevet EP 300099, EP 529715, EP 621282 et EP 649854 ainsi qu'aux documents C. van Boeckel, M Petitou, Angew. Chem. Int. Ed. Engl., 1993, 32, 1671-1690. Ces synthons sont ensuite couplés les uns aux autres de façon à fournir un équivalent entièrement protégé d'un polysaccharide selon l'invention. Cet équivalent protégé est ensuite transformé en un composé selon l'invention.

L'un des synthons de base évoqués ci-dessus contient une fonction protégée particulière permettant l'introduction ultérieure de la biotine ou d'un dérivé de biotine, par exemple, une fonction amine latente sous forme de groupe azido ou protégée sous forme de N-phtalimido.

Dans les réactions de couplage évoquées ci-dessus, un di- ou oligosaccharide "donneur", activé sur son carbone anomère, réagit avec un di- ou oligosaccharide "accepteur", possédant un hydroxyle libre.

La présente invention concerne un procédé pour la préparation des composés de formule (I) caractérisé en ce que : dans une première étape, un équivalent complètement protégé du polysaccharide (I) désiré, contenant un précurseur protégé du domaine Pe prolongé à son extrémité non réductrice par un précurseur protégé du polysaccharide sulfaté Po est synthétisé, l'un de ces précurseurs contient notamment une fonction amine convenablement protégée pour l'introduction ultérieure de la biotine ou d'un dérivé de biotine ; dans une seconde étape, les groupes chargés négativement sont introduits et/ou démasqués ; dans une troisième étape, on déprotège la fonction amine puis on introduit la biotine ou le dérivé de biotine.

La synthèse de Pe est réalisée selon les méthodes décrites, en particulier dans les demandes de brevet publiées sous les numéros WO98/03554 et WO99/36443 ainsi que dans la littérature (citée ci-dessus).

La synthèse de la partie polysaccharidique précurseur de Po est réalisée selon des réactions bien connues de l'homme de l'art, en utilisant les méthodes de la synthèse d'oligosaccharides (G.J. Boons, Tetrahedron, 1996, 52, 1095-1121, WO98/03554 et

WO99/36443) ou un oligosaccharide lorsqu'un oligosaccharide donneur de liaison glycosidique est couplé avec un oligosaccharide accepteur de liaison glycosidique pour conduire à un autre oligosaccharide dont la taille est égale à la somme des tailles des deux espèces réactives. Cette séquence est répétée jusqu'à l'obtention du composé de formule (I) désiré. La nature et le profil de la charge du composé final désiré déterminent la nature des entités chimiques utilisées dans les différentes étapes de la synthèse, selon les règles bien connues de l'homme de l'art. On pourra se référer par exemple à C. van Boeckel, M. Petitou, Angew. Chem. Int. Ed. Engl. 1993, 32, 1671-1690 ou encore à H. Paulsen, "Advances in selective chemical syntheses of complex oligosaccharides" Angew. Chem. lnt. Ed. Engl. 21, 155-173 (1982).

Les composés de l'invention sont obtenus à partir de leurs précurseurs polysaccharidiques complètement protégés en utilisant l'enchaînement suivant de réactions :
- les fonctions alcool devant être transformées en un groupe O-sulfo, et les acides carboxyliques sont déprotégés par l'élimination des groupes protecteurs utilisés durant l'élaboration du squelette puis,
- les groupes sulfo sont ensuite introduits,
- la fonction amine permettant d'introduire la biotine ou le dérivé de biotine est déprotégée,
- la biotine ou le dérivé de biotine est introduite par une réaction classique de couplage amino / acide.

Les composés de l'invention peuvent naturellement être préparés en utilisant différentes stratégies connues par l'homme de l'art de la synthèse des oligosaccharides.

Le procédé décrit ci-dessus est le procédé préféré de l'invention. Toutefois, les composés de formule (I) peuvent être préparés par d'autres méthodes bien connues de la chimie des sucres décrites par exemple dans Monosaccharides, Their chemistry and their roles in natural products, P.M. Collins et R.J. Ferrier, J. Wiley & Sons, 1995 et dans G.J. Boons, Tetrahedron, 1996, 52, 1095-1121.

Les pentasaccharides Pe peuvent donc être obtenus à partir de synthons disaccharidiques de la façon décrite dans la publication de C. van Boeckel, M. Petitou, Angew. Chem. lnt. Ed. Engl. 1993, 32,1671-1690.

Les groupes protecteurs utilisés dans le procédé de préparation des composés (I) sont ceux couramment utilisés dans la chimie des sucres, par exemple dans Protective Groups in Organic Synthesis, TW Greene, John Wiley & sons, New-York, 1981.

Les groupes protecteurs sont avantageusement choisis, par exemple parmi les groupes acétyles, halogénométhyles, benzoyles, lévulinyles, benzyles, benzyles substitués, trityles éventuellement substitués, tétrahydropyranyles, allyles, pentenyles, *tert*-butyldiméthylsilyles (tBDMS) ou triméthylsilyléthyles.

Les groupes activateurs sont ceux classiquement utilisés en chimie des sucres selon par exemple G.J. Boons, Tetrahedron, 1996, 52, 1095-1121. Ces groupes activateurs sont choisis par exemple parmi les imidates, les thioglycosides, les penténylglycosides, les xanthates, les phosphites ou les halogénures.

En ce qui concerne la façon dont la biotine est liée à l'oligosaccharide ainsi que la nature du dérivé de biotine, la littérature chimique offre d'autres possibilités qu'il est possible de mettre en oeuvre par des jeux de groupes protecteurs bien connus de l'homme de l'art. On utilisera de préférence une fonction amine, ou encore une fonction thiol, ou encore une fonction acide carboxylique ou encore une fonction aldéhyde que l'on fera réagir avec un dérivé de biotine comportant un groupe réactif du type ester activé, maléimide, iodoacétyl ou amine primaire, la réaction se faisant selon les conditions décrites dans la littérature (cf. Savage et al., Avidin-Biotin Chemistry: A Handbook, Pierce Chemical Company, 1992).

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir le sel souhaité. Pour la préparation des sels des composés de formule (I), on peut utiliser toute base minérale ou organique, donnant avec les composés de formule (I), des sels pharmaceutiquement acceptables. On utilise de manière préférentielle comme base l'hydroxyde de sodium, de potassium, de calcium ou de magnésium. Les sels de sodium et de calcium des composés de formule (I), sont les sels préférés.

Les composés selon l'invention ont fait l'objet d'études biochimiques et pharmacologiques.

L'activité antithrombotique globale de ces produits et leur neutralisation a été étudiée dans un modèle de thrombose veineuse constitué d'une injection de facteur tissulaire suivie d'une stase de la veine cave de rats, tels que décrits par J.-M. Herbert et al., Blood, 1998, 91, 4197-4205. Dans ce modèle, une inhibition de 60 % de la thrombose est obtenue après injection intraveineuse de 0,1 à 30 mmol / kg des composés.

L'injection d'avidine dans un rapport molaire de 1 à 1000 réduit fortement l'effet antithrombotique de ces composés, la réduction obtenue pouvant être supérieure à 50%. Parallèlement, l'activité pro hémorragique des composés est neutralisée par l'injection d'avidine aux doses précitées. De même, l'activité circulante des oligosaccharides, mesurée par l'activité anti-Xa et / ou l'activité anti-IIa, est neutralisée par l'injection d'avidine.

Ainsi, la présente invention a également pour objet un procédé mettant en oeuvre l'avidine ou la streptavidine caractérisé en ce qu'il permet de neutraliser les polysaccharides selon l'invention. L'avidine ou la streptavidine peuvent être utilisées pour la préparation de médicaments destinés à neutraliser les polysaccharides selon la présente invention.

Grâce à leur activité biochimique et pharmaceutique, les oligosaccharides de la présente invention constituent des médicaments très intéressants. Leur toxicité est parfaitement compatible avec cette utilisation. Ils sont également très stables et sont donc particulièrement appropriés pour constituer le principe actif de spécialités pharmaceutiques.

Ils peuvent être utilisés dans diverses pathologies consécutives à une modification de l'homéostasie du système de la coagulation apparaissant en particulier lors des troubles du système cardio-vasculaire et cérébro-vasculaire comme les troubles thromboemboliques associés à l'arthérosclérose et au diabète tels que l'angine instable, l'attaque cérébrale, la resténose après angioplastie, l'endartérectomie, la pose de prothèses endovasculaires ; ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens.

Ces produits peuvent par ailleurs être utilisés pour le traitement ou la prévention de pathologies thromboemboliques d'origine veineuse telles les embolies pulmonaires. Ils peuvent être utilisés ou pour prévenir ou pour traiter les complications thrombotiques observés par exemple à la suite d'opérations chirurgicales, de développements tumoraux ou de dérèglements de la coagulation, induits par des activateurs bactériens, viraux, ou enzymatiques. Dans le cas de leur utilisation lors de la pose de prothèses, les composés de la présente invention peuvent recouvrir des prothèses et les rendre ainsi hémocompatibles. En particulier, ils peuvent être fixés à des prothèses intravasculaires (stents). Dans ce cas, ils peuvent éventuellement être modifiés chimiquement par introduction à l'extrémité non réductrice ou réductrice d'un bras approprié, comme décrit selon EP 649854.

Les composés de la présente invention peuvent également être utilisés comme adjuvants lors d'endartérectomie réalisée avec des ballonnets poreux.

Les composés selon l'invention peuvent être utilisés pour la préparation de médicaments destinés à traiter les maladies ci-dessus.

Selon un autre de ses aspects, la présente invention a donc pour objet une composition pharmaceutique contenant, en tant que principe actif, un polysaccharide de synthèse selon l'invention ou un de ses sels pharmaceutiquement acceptables, éventuellement en association avec un ou plusieurs excipients inertes et appropriés.

Lesdits excipients sont choisis selon la forme pharmaceutique et le mode d'administration souhaités : orale, sublinguale, sous-cutanée, intramusculaire, intraveineuse, transdermique, transmuqueux, locale ou rectale.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

Le principe actif peut également être libéré par un ballonnet le contenant ou par un extenseur endovasculaire introduit dans les vaisseaux sanguins. L'efficacité pharmacologique du principe actif n'est ainsi pas affectée.

Dans chaque unité de dosage, le principe actif est présent dans les quantités adaptées aux doses journalières envisagées afin d'obtenir l'effet prophylactique ou thérapeutique désiré. Chaque unité de dosage peut contenir de 0,1 à 100 mg de principe actif, de préférence 0,5 à 50 mg. Ces doses de composés anticoagulants pourraient être neutralisées par des doses d'avidine ou de streptavidine allant de 1 à 1000 mg en injection iv (intraveineuse) bolus ou perfusion

Les composés selon l'invention peuvent également être utilisés en association avec un ou plusieurs autres principes actifs utiles pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoprotéine IIb/IIIa.

Les méthodes, les préparations et les schémas suivants illustrent la synthèse des différents intermédiaires utiles à l'obtention des polysaccharides selon l'invention.

Les abréviations suivantes sont utilisées :
Bn : benzyle ; Bz : benzoyle ; CCM : chromatographie sur couche mince ; Ts : tosyle ; Lev : lévulinyl ; Et : éthyle ; Ph : phényle ; Me : méthyle ; Ac : acétyle ; SE : triméthylsilyléthyl ; ESI : est le sigle anglais Electron Spray lonisation ; Biotine : Acide hexahydro-2oxo-1*H*-thiéno[3,4-d]imidazole-4-pentanoïque ; Z : benzyloxycarbonyle.

Dans la suite, des exemples de synthèse des composés de l'invention sont détaillés à titre d'illustration.

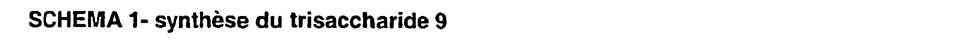

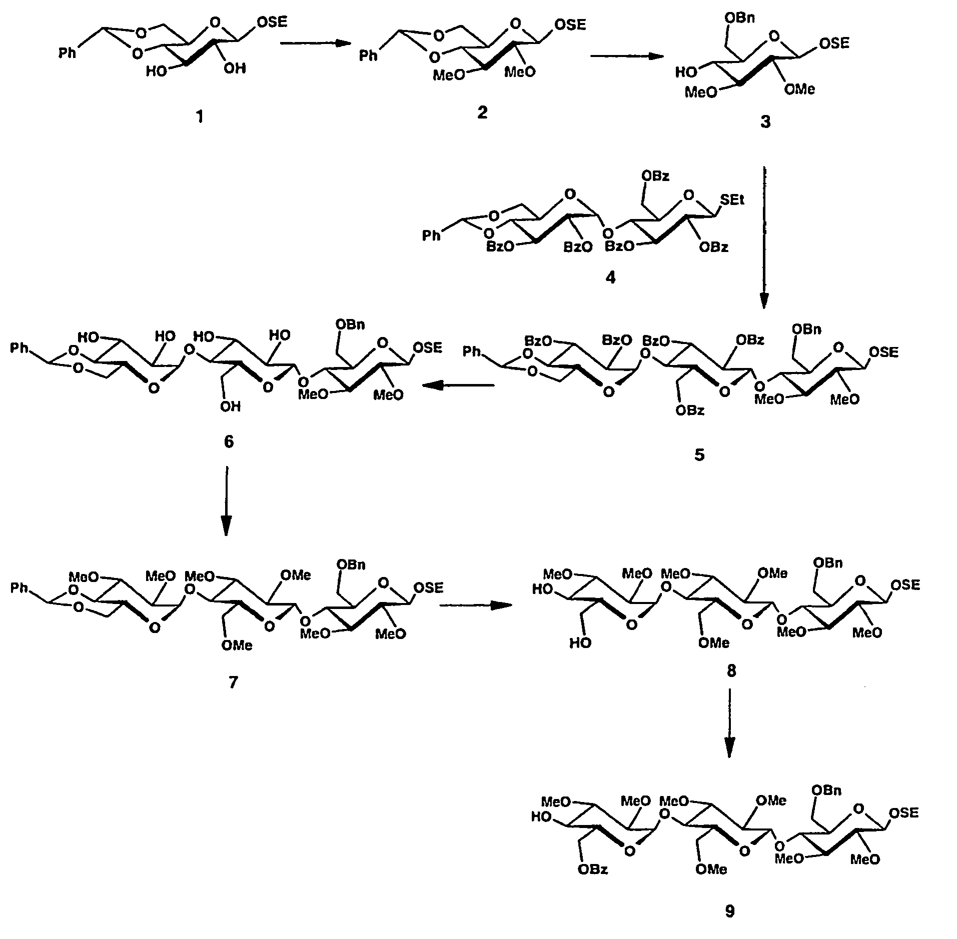

### PREPARATION 1

### 2-(Triméthylsilyl)éthyl 4,6-O-benzylidène-2,3-di-O-méthyl-O-D-glucopyranoside (2)

A une solution du composé 1 (15,8 g, 42,8 mmol) (préparé selon K. Jansson et al., J. Org. Chem., 1988, 53, 5629-5647) et d'iodure de méthyle (20 mL, 319 mmol) dans le tétrahydrofurane (350 mL), on ajoute, à 0 °C, par petites quantités de l'hydrure de sodium (18 g). On agite pendant 4 heures le mélange réactionnel à température ambiante. L'excès d'hydrure de sodium est détruit avec du méthanol et le mélange réactionnel est versé dans l'eau glacée (1,5 L). Après extraction avec de l'acétate d'éthyle, la phase organique est lavée avec une solution saturée de chlorure de sodium, de l'eau, séchée sur sulfate de sodium puis concentrée sous vide. La purification du résidu est effectuée par chromatographie sur colonne de gel de silice (cyclohexane/acétate d'éthyle 15/1 (v/v)) pour donner 16,8 g du composé 2.
[α]_{D} = -41° (c = 0,69, dichlorométhane).

### PREPARATION 2

### 2-(Triméthylsilyl)éthyl 6-O-benzyl-2,3-di-O-méthyl-β-D-glucopyranoside (3)

A une solution du composé 2 (16 g, 40,3 mmol) dans le tétrahydrofurane (600 mL), on ajoute successivement du tamis moléculaire 3Å en poudre (82 g), de l'orange de méthyle (indicateur coloré), du cyanoborohydrure de sodium (34 g, 526 mmol) puis goutte à goutte une solution saturée d'acide chlorhydrique dans du diéthyléther jusqu'à obtenir une coloration rose. Après filtration et extraction avec de l'acétate d'éthyle, la phase organique est lavée avec une solution saturée d'hydrogénocarbonate de sodium, de l'eau, séchée sur sulfate de sodium puis concentrée sous vide. Une chromatographie sur colonne de gel de silice (toluène/acétate d'éthyle 3/1 (v/v)) permet d'obtenir 12,5 g du composé 3.
[α]_{D} = -42° (c = 1,2, dichlorométhane).

### PREPARATION 3

### 2-(Triméthylsilyl)éthyl (2,3-di-O-benzoyl-4,6-O-benzylidène-α-D-glucopyranosyl)-(1→4)-(2,3,6-tri-O-benzoyl-β-D-glucopyranosyl)-(1→4)-6-O-benzyl-2,3-di-O-méthyl-β-D-glucopyranoside (5)

Un mélange de thioglycoside 4 (16,52 g, 16,60 mmol) (obtenu selon la préparation 1 de la demande de brevet publiée sous le numéro WO 99/36443), du composé 3 (6,0 g, 15,05 mmol) et de tamis moléculaire 4Å en poudre (16,7 g) dans le toluène (300 mL) est agité sous atmosphère d'argon pendant 1 heure. Puis le mélange est refroidi à - 20 C. Une solution de *N*-iodosuccinimide (3,9 g, 17,4 mmol) et d'acide trifluorométhane sulfonique (0,17 mL, 1,97 mmol) dans un mélange dichlorométhane/dioxane 1/1 (v/v) (86 mL) est ajoutée goutte à goutte au mélange réactionnel. Après 10 minutes, le mélange réactionnel est filtré, dilué avec du dichlorométhane, successivement lavé avec une solution de thiosulfate de sodium 1 M, une solution d'hydrogénocarbonate de sodium à 10% et de l'eau, séché sur sulfate de sodium puis concentré sous vide. La purification du résidu est effectuée par chromatographie sur colonne de gel de silice (toluène/acétate d'éthyle 6/1 (v/v)) pour donner 18,8 g du trisaccharide 5.
[α]_{D} = +34° (c = 1,26, dichlorométhane).

### PREPARATION 4

### 2-(Triméthylsilyl)éthyl (4,6-O-benzylidène-α-D-glucopyranosyl)-(1→4)-(β-D-glucopyranosyl)-(1→4)-6-O-benzyl-2,3-di-O-méthyt-β-D-glucopyranoside (6)

A une solution du composé 5 (18,7 g, 14 mmol) dans un mélange méthanol-dioxane 1/1 (v/v) (140 mL) est ajouté du *tert*-butylate de potassium (3,15 g). On agite pendant 2 heures à température ambiante. Le mélange réactionnel est neutralisé avec de la résine Dowex^{®} 50WX4 H⁺, filtré et concentré sous vide. La purification du résidu est effectuée par chromatographie sur colonne de gel de silice (dichlorométhane/méthanol 20/1 (v/v)) pour donner 10,0 g du composé 6.
[α]_{D} = +29° (c = 1,11, dichlorométhane).

### PREPARATION 5

### 2-(Triméthylsilyl)éthyl (4,6-O-benzylidène-2,3-di-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (7)

A un mélange refroidi (0°C) du composé 6 (9,93 g, 12,24 mmol), d'iodure de méthyle (9,0 mL, 138 mmol) dans du tétrahydrofurane anhydre (100 mL), on ajoute, par petites quantités, de l'hydrure de sodium (5.2 g, 216 mmol) sous atmosphère d'argon. Le mélange est agité pendant 20 heures à température ambiante. L'excès d'hydrure de sodium est détruit avec du méthanol et le mélange réactionnel est versé dans l'eau glacée (500 mL). Après extraction avec de l'acétate d'éthyle, la phase organique est lavée avec une solution saturée de chlorure de sodium, séchée sur sulfate de sodium puis concentrée sous vide pour donner 11 g du composé 7 qui est utilisé dans l'étape suivante sans purification.
CCM : Rf = 0,38, gel de silice, toluène/acétate d'éthyle 3/2 (v/v)

### PREPARATION 6

### 2-(Triméthylsilyl)éthyl (2,3-di-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (8)

Le composé 7 (11 g) est dissous dans de l'acide acétique à 60% (180 mL) et agité pendant 1 heure 30 à 80 °C. Le mélange est concentré et co-évaporé avec du toluène. Le résidu est purifié par chromatographie sur colonne de gel de silice (toluène/acétone 2/1 (v/v)) pour donner 8,46 g du composé 8.
CCM : Rf = 0,36, gel de silice, toluène/acétone 1/1 (v/v)

### PREPARATION 7

### 2-(Triméthylsilyl)éthyl (6-O-benzoyl-2,3-di-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (9)

A une solution du composé 8 (8,41 g, 10,6 mmol) dans du dichlorométhane (110 mL), on ajoute du 1-benzoyloxy-1*H*-benzotriazole (5,36 g, 22,4 mmol) et de la triéthylamine (3,32 mL). Le mélange est agité pendant 20 heures à température ambiante, puis dilué avec du dichlorométhane, lavé avec une solution saturée d'hydrogénocarbonate de sodium et de l'eau, séché sur sulfate de sodium puis concentré sous vide. Le résidu est purifié par chromatographie sur colonne de gel de silice (cyclohexane/acétate d'éthyle/éthanol 5/0,5/0,25 (v/v/v)) pour donner 8,40 g du composé 9.
[α]_{D} = +15° (c = 2, dichlorométhane).

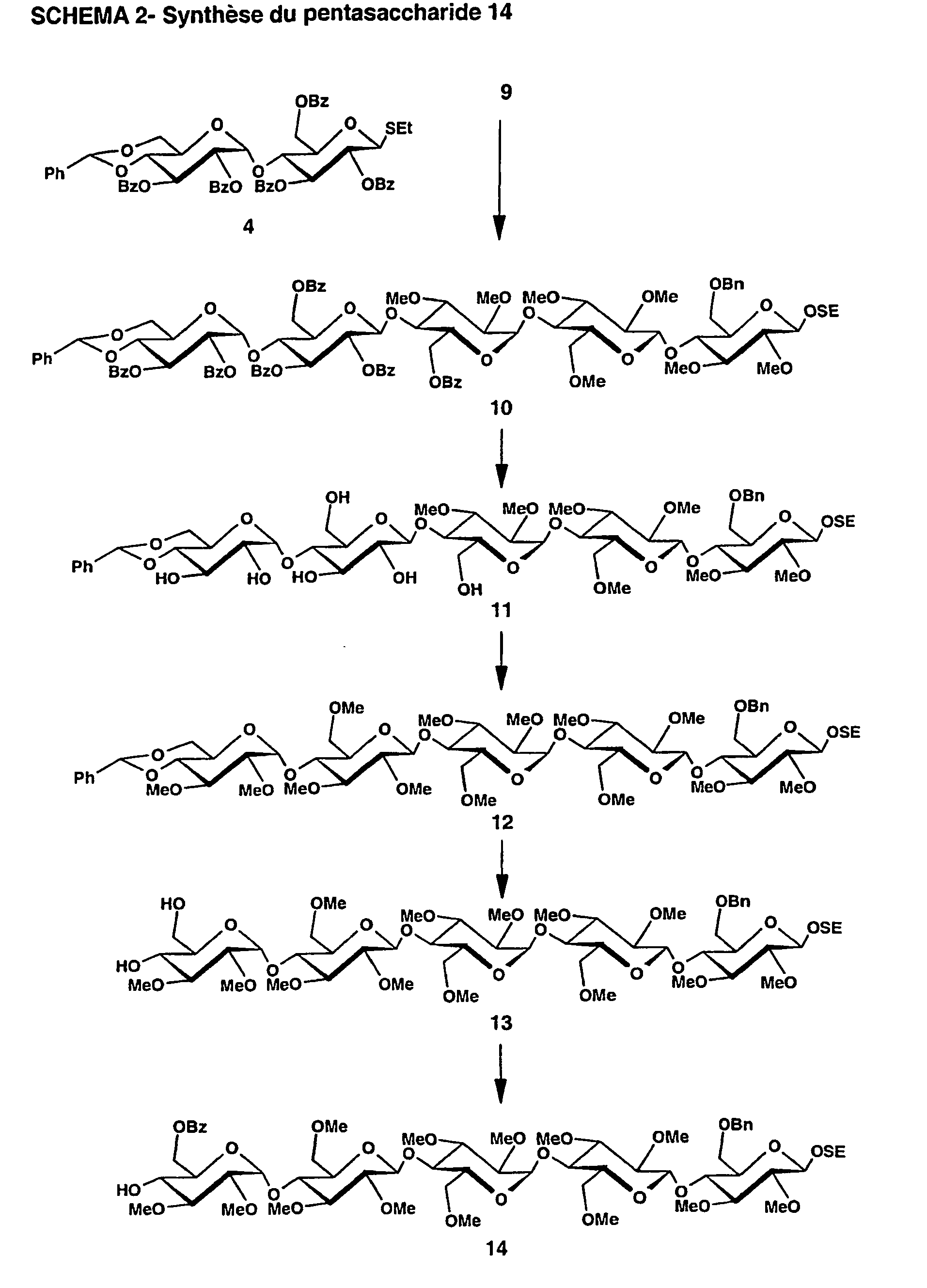

### PREPARATION 8

### 2-(Triméthyisilyl)éthyl (2,3-di-O-benzoyl-4,6-O-benzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (10)

Le composé 9 est transformé en composé 10 selon le mode opératoire décrit à la PREPARATION 3.
[α]_{D} = +42° (c = 2, dichlorométhane).

### PREPARATION 9

### 2-(Triméthylsilyl)éthyl (4,6-O-benzylidène-α-D-glucopyranosyl)-(1→4)-(β-D-gluco-pyranosyl)-(1→4)-(2,3-di-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (11)

Le composé 10 est transformé en composé 11 selon le mode opératoire décrit à la PREPARATION 4.
CCM : Rf= 0,35, gel de silice, dichlorométhane/méthanol 10/1 (v/v)

### PREPARATION 10

### 2-(Triméthylsilyl)éthyl (4,6-O-benzylidène-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (12)

Le composé 11 est transformé en composé 12 selon le mode opératoire décrit à la PREPARATION 5.
CCM : Rf = 0,11, gel de silice, cyclohexane/acétate d'éthyle 1/2 (v/v)

### PREPARATION 11

### 2-(Triméthylsilyl)éthyl (2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (13)

Le composé 12 est transformé en composé 13 selon le mode opératoire décrit à la PREPARATION 6.
CCM : Rf = 0,33, gel de silice, cyclohexane/acétate d'éthyle/éthanol 2/0,5/0,5 (v/v/v)

### PREPARATION 12

### 2-(Triméthylsilyl)éthyl (6-O-benzoyl-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (14)

Le composé 13 est transformé en composé 14 selon le mode opératoire décrit à la PREPARATION 7.
CCM : Rf = 0,16, gel de silice, cyclohexane/acétate d'éthyle/éthanol 3/0,5/0,5 (v/v/v)

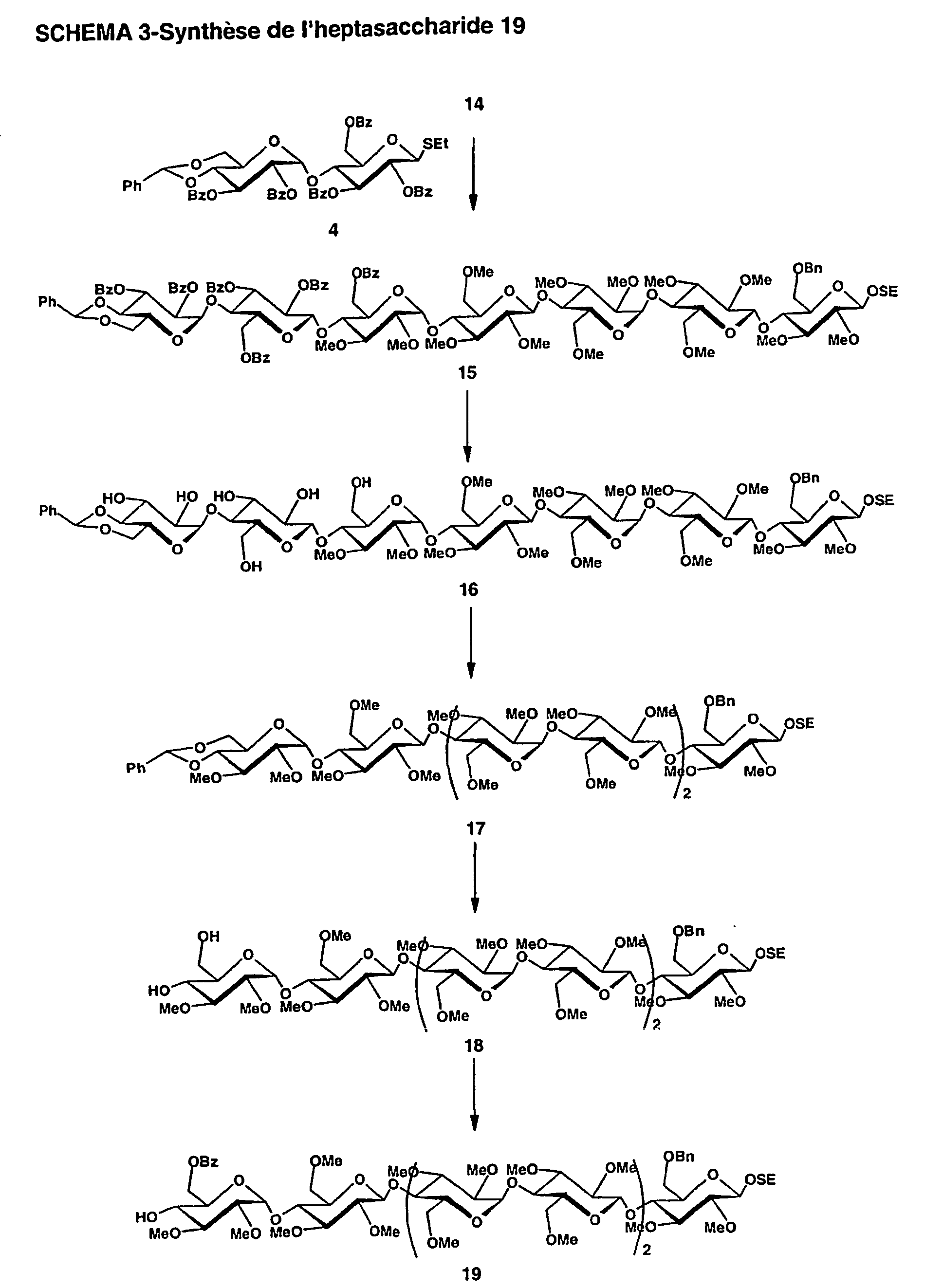

### PREPARATION 13

### 2-(Triméthylsilyl)éthyl (2,3-di-O-benzoyl-4,6-O-bonzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-O-glucopyranoside (15)

La réaction de couplage du composé 14 avec le disaccharide 4 est réalisée selon le mode opératoire décrit à la PREPARATION 3, pour fournir le composé 15.
[α]_{D} = +52° (c = 1,1, dichlorométhane).

### PREPARATION 14

### 2-(Triméthylsilyl)éthyl(4,6-O-benzylidène-α-D-glucopyranosyl)-(1→4)-(β-D-glucopyranosyl)-(1→4)-(2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (16)

Le composé 15 est transformé en composé 16 selon le mode opératoire décrit à la PREPARATION 4.
CCM : Rf = 0,31, gel de silice, dichlorométhane/méthanol 10/1 (v/v)

### PREPARATION 15

### 2-(Triméthylsilyl)éthyl (4,6-O-benzylidène-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₂-6-O-benzyl-2,3-di-O-méthyl-β-D-glucopyranoside (17)

Le composé 16 est transformé en composé 17 selon le mode opératoire décrit à la PREPARATION 5.
CCM : Rf = 0,46, gel de silice, dichlorométhane/méthanol 10/1 (v/v)

### PREPARATION 16

### 2-(Triméthylsilyl)éthyl (2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (18)

Le composé 17 est transformé en composé 18 selon le mode opératoire décrit à la PREPARATION 6.
CCM : Rf = 0,42, gel de silice, cyclohexane/acétate d'éthyle/éthanol 1/0,5/0,5 (v/v/v)

### PREPARATION 17

### 2-(Triméthylsilyl)éthyl (6-O-benzoyl-2,3-di-O-méthyl-α-D-glucopyranosyl)-(1→4)-(2,3,6-tri-O-méthyl-β-D-glucopyranosyl)-(1→4)-(2,3,6-tri-O-méthyl-α-D-glucopyranosyl)-(1→4)-(2,3,6-tri-O-méthyl-β-O-glucopyranosyl)-(1→4)-(2,3,6-tri-O-méthyl-α-D-glucopyranosyl)-(1→4)-(2,3,6-tri-O-méthyl-β-D-glucopyranosyl)-(1→4)-6-O-benzyl-2,3-di-O-méthyl-β-D-glucopyranoside (19)

Le composé 18 est transformé en composé 19 selon le mode opératoire décrit à la PREPARATION 7.
CCM : Rf = 0,25, gel de silice, cyclohexane/acétate d'éthyle/éthanol 3/0,5/0,5 (v/v/v)

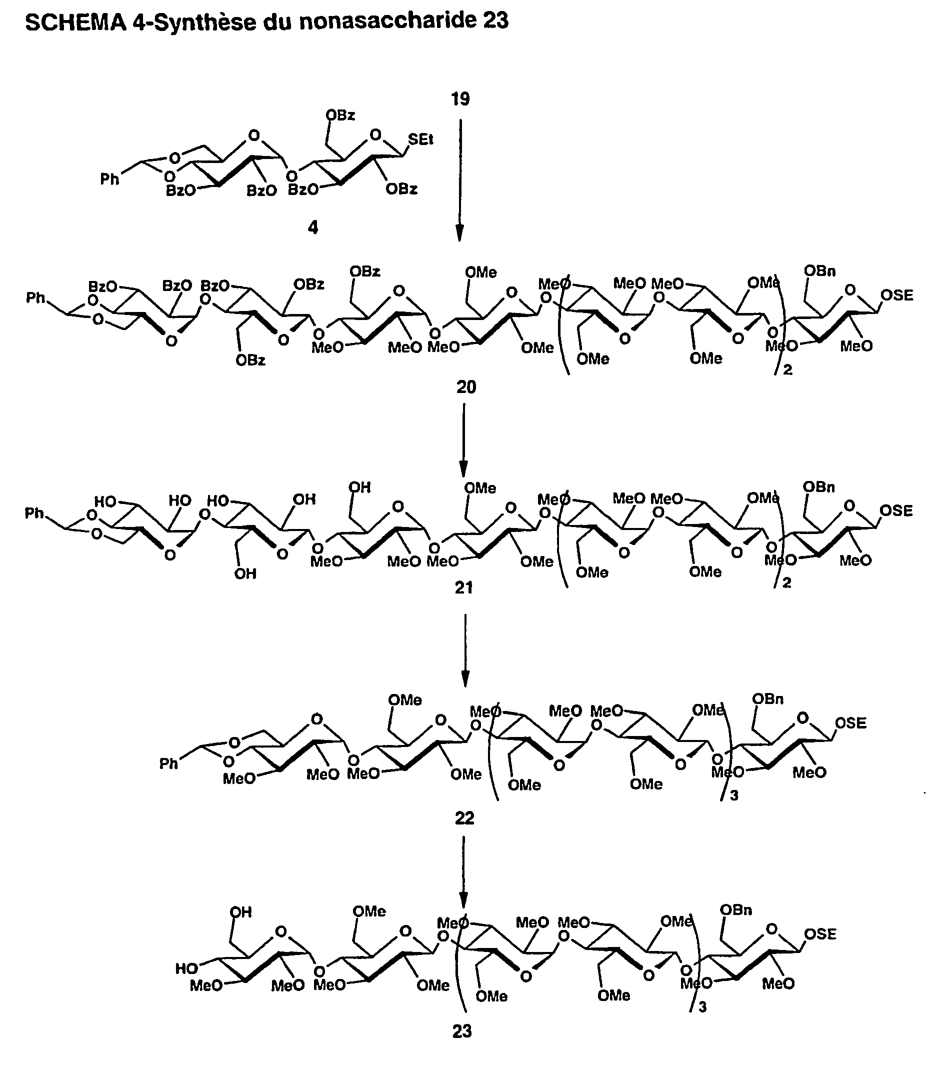

### PREPARATION 18

### 2-(Triméthylsilyl)éthyl (2,3-di-O-benzoyl-4,6-O-benzylidène-α-D-glucopyranosyl)-(1→4)-(2,3,6-tri-O-benzoyl-β-D-glucopyranosyl)-(1→4)-(6-O-benzoyl-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₂-6-O-benzyl-2,3-di-O-méthyl-β-D-glucopyranoside (20)

Un mélange du thioglycoside 4 (5,2 g, 5,23 mmol), d'heptasaccharide 19 (4,72 g, 2,75 mmol) et de tamis moléculaire 4Å en poudre dans le toluène est agité sous atmosphère d'argon pendant 1 heure. On ajoute alors goutte à goutte, à 0°C, une solution de *N*-iodosuccinimide (1,36 g, 5,85 mmol) et d'acide trifluorométhanesulfonique (0,140 mL, 1,56 mmol) dans du dichlorométhane/dioxane 1/1 (v/v) (32 mL). Après 15 minutes, le mélange réactionnel est filtré, dilué avec du dichlorométhane, lavé successivement avec une solution de thiosulfate de sodium 1 M, une solution d'hydrogénocarbonate de sodium à 10% et de l'eau, séché sur sulfate de sodium puis concentré sous vide. La purification du résidu est effectuée par chromatographie sur colonne de gel de silice (cyclohexane/acétate d'éthyle/éthanol 3/0,5/0,5 (v/v/v)) pour donner 7,13 g du composé 20.
[α]_{D} = +65° (c = 1,4, dichlorométhane).

### PREPARATION 19

### 2-(Triméthylsilyl)éthyl (4,6-O-benzylidène-α-D-glucopyranosyl)-(1→4)-(β-D-glucopyranosyl)-(1→4)-(2,3-di-O-méthyl-α-D-glucopyranosyl)-(1→4)-(2,3,6-tri-O-methyl-β-D-glucopyranosyl)-(1→4)-[(2,3,6-tri-O-méthyl-α-O-glucopyranosyl)-(1→4)-(2,3,6-tri-O-méthyl-β-D-glucopyranosyl)-(1→4)]₂-6-O-benzyl-2,3-di-O-méthyl-β-O-glucopyranoside (21)

Le composé 20 est transformé en composé 21 selon le mode opératoire décrit à la PREPARATION 4.
CCM : Rf = 0,27, gel de silice, dichlorométhane/méthanol 10/1 (v/v)

### PREPARATION 20

### 2-(Triméthylsilyl)éthyl (4,6-O-bonzylidène-2,3-di-O-méthyl-α-D-glucopyranosyl)-(1→4)-(2,3,6-tri-O-méthyl-β-D-glucopyranosyl)-(1→4)-[(2,3,6-tri-O-méthy-α-D-glucopyranosyl)-(1→4)-(2,3,6-tri-O-méthyl-β-D-glucopyranosyl)-(1→4)]₃-6-O-benzyl-2,3-di-O-méthyl-β-D-glucopyranoside (22)

Le composé 21 est transformé en composé 22 selon le mode opératoire décrit à la **PREPARATION 5.**
CCM : Rf = 0,31, gel de silice, cyclohexane/acétate d'éthyle/éthanol 5/1/1 (v/v/v)

### PREPARATION 21

### 2-(Triméthylsilyl)éthyl (2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3, 6-tri-O-méthyl-β-D-glucopyranosyl)-(1→4)]₃-6-O-benzyl-2,3-di-O-méthyl-β-D-glucopyranoside (23)

Le composé 22 est transformé en composé 23 selon le mode opératoire décrit à la PREPARATION 6.
CCM : Rf = 0,35, gel de silice, cyclohexane/acétate d'éthyle/éthanol 2/1/1 (v/v/v)

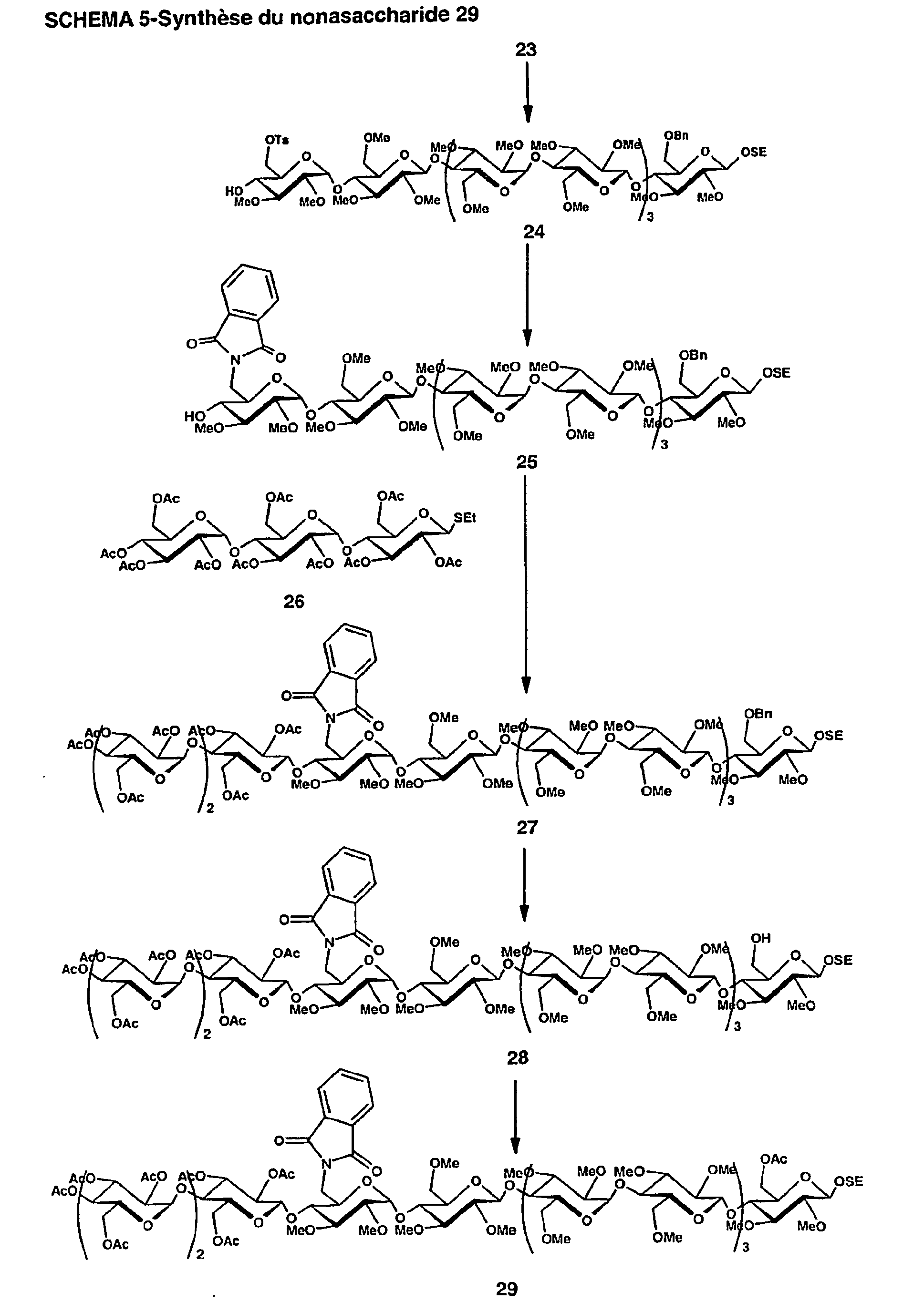

### PREPARATION 22

### 2-(Triméthylsilyl)éthyl (2,3-di-O-méthyl-6-O-tosyl-α-D-glucopyranosyl)-(1→4)-(2,3,6-tri-O-méthyl-O-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-6-O-benzyl-2,3-di-O-méthyl-β-D-glucopyranoside (24)

Sous atmosphère d'argon, le composé 23 (1,09 g) est dissous dans la pyridine (10 mL), puis du chlorure de tosyle (1,03 g) est ajouté. Après 2 heures d'agitation, on dilue avec du dichlorométhane (100 mL). La phase organique est lavée successivement avec une solution d'hydrogénosulfate de potassium à 10% puis de l'eau, séchée et évaporée à sec. Après chromatographie sur colonne de gel de silice (toluène/acétone 2,3/2 (v/v)), on obtient 1,77 g du composé 24.
CCM : Rf = 0,5, gel de silice, cyclohexane/acétate d'éthyle/éthanol 3/1/1 (v/v/v)

### PREPARATION 23

### 2-(Triméthylsilyl)éthyl (6-désoxy-2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-6-O-benzyl-2,3-di-O-méthyl-β-D-glucopyranoside (25)

A une solution du composé 24 (500 mg, 0,23 mmol) dans du *N,N*-diméthylformamide anhydre (11 mL) contenant du tamis moléculaire 4Å en poudre, on ajoute du phthalimide de potassium (225 mg, 1,38 mmol) puis de l'éther couronne 18-crown-6 (121,5 mg, 0,46 mmol). Le mélange est agité pendant 4 heures à 80°C. Après avoir été refroidi, le mélange réactionnel est dilué avec du dichlorométhane, filtré sur Célite et concentré. Le résidu est purifié par chromatographie sur gel Sephadex^{®} LH20 (3 x 100 cm) (dichlorométhane/thanol 1/1 (v/v)) suivi par une chromatographie sur colonne de gel de silice (toluène/éthanol 11/2 (v/v)) pour donner 417,4 mg du composé 25.
CCM : Rf = 0,38, gel de silice, toluène/éthanol 11/2 (v/v)

### PREPARATION 24

### 2-(Triméthylsilyl)éthyl (2,3,4,6-tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-désoxy-2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-O-glucopyranosyl)-(1→4)]3-6-O-benzyl-2,3-di-O-méthyl-β-D-glucopyranoside (27)

Un mélange de thioglycoside 26 (1,515 g, 1,564 mmol) (préparé selon le composé 41, préparation 36 du brevet WO 99/36443), de l'accepteur 25 (840 mg, 0,391 mmol) et de tamis moléculaire 4Å en poudre (2,15 g) dans le toluène (33 mL) est agité sous atmosphère d'argon pendant 1 heure. Le mélange réactionnel est refroidi à 0°C et une solution de *N*-iodosuccinimide (387 mg) et d'acide trifluorométhanesulfonique (55,4 µL) dans le dichlorométhane/dioxane 1/1 (v/v) (7 mL) y est introduite. Après 10 minutes, le mélange est filtré, dilué avec du toluène, lavé successivement avec une solution de thiosulfate de sodium 1 M, une solution d'hydrogénocarbonate de sodium à 10% et de l'eau, séché sur sulfate de sodium puis concentré sous vide. La purification du résidu est faite par chromatographie sur gel Sephadex® LH20 (dichlorométhane/éthanol 1/1 (v/v)), suivie par chromatographie sur colonne de gel de silice (toluène/acétone 5/4 (v/v)) et conduit à 887 mg du composé 27.
[α]_{D} = +70° (c = 0,35, dichlorométhane).

### PREPARATION 25

### 2-(Triméthylsilyl)éthyl (2,3,4,6-tétra-O-acétyl-α-D-glucopyranosyl)-(1→4)-(2,3,6-tri-O-acétyhc-α-D-glucopyranosyl)-(1→4)-(2,3,6-tri-O-acétyl-β-D-glucopyranosyl)-(1→4)-(6-désoxy-2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-2,3-di-O-méthyl-β-D-glucopyranoside (28)

Une solution du composé 27 (750 mg, 0,245 mmol) dans de l'acide acétique (37 mL) est traitée sous pression d'hydrogène (5 bars) en présence de palladium sur charbon 10% (750 mg) pendant 2 heures 30. Après filtration, la solution est concentrée et la purification du résidu est effectuée par chromatographie sur colonne de gel de silice (toluène/éthanol 6/1 (v/v)) pour donner 728 mg du composé 28.
CCM : Rf = 0,32, gel de silice, toluène/éthanol 6/1 (v/v)

### PREPARATION 26

### 2-(Triméthylsilyl)éthyl (2,3,4,6-tétra-O-acétyl-α-D-glucopyranosyl)-(1→4)-(2,3,6-triO-acétyl-α-D-glucopyranosyl)-(1→4)-(2,3,6-tri-O-acétyl-β-D-glucopyranosyl)-(1→4)-(6-désoxy-2,3-di-O-méthyl-6-phthalimido-α-D-glucopyranosyl)-(1→4)-(2,3,6-tri-O-méthyl-β-O-glucopyranosyl)-(1→4)-[(2,3,6-tri-O-méthyl-α-D-glucopyranosyl)-(1→4)-(2,3,6-tri-O-méthyl-β-D-glucopyranosyl)-(1→4)]₃-6-O-acétyl-2,3-di-O-méthyl-β-D-glucopyranoside (29)

A une solution du composé 28 (728 mg, 0,245 mmol) dans le dichlorométhane (10 mL) on ajoute de la triéthylamine (51,1 µL, 0,368 mmol), de l'anhydride acétique (32,5 µL, 0,344 mmol) et de la diméthylaminopyridine (6,0 mg, 0,049 mmol). Après 1 heure d'agitation, on dilue avec du dichlorométhane, on lave successivement avec une solution d'hydrogénosulfate de potassium à 10%, de l'eau, une solution saturée d'hydrogénocarbonate de sodium et de l'eau, séché sur sulfate de sodium puis concentré sous vide. La purification du résidu effectuée par chromatographie sur colonne de gel de silice permet d'obtenir 0,618 mg du composé 29.
CCM : Rf = 0,37, gel de silice, toluène/éthanol 6/1 (v/v)

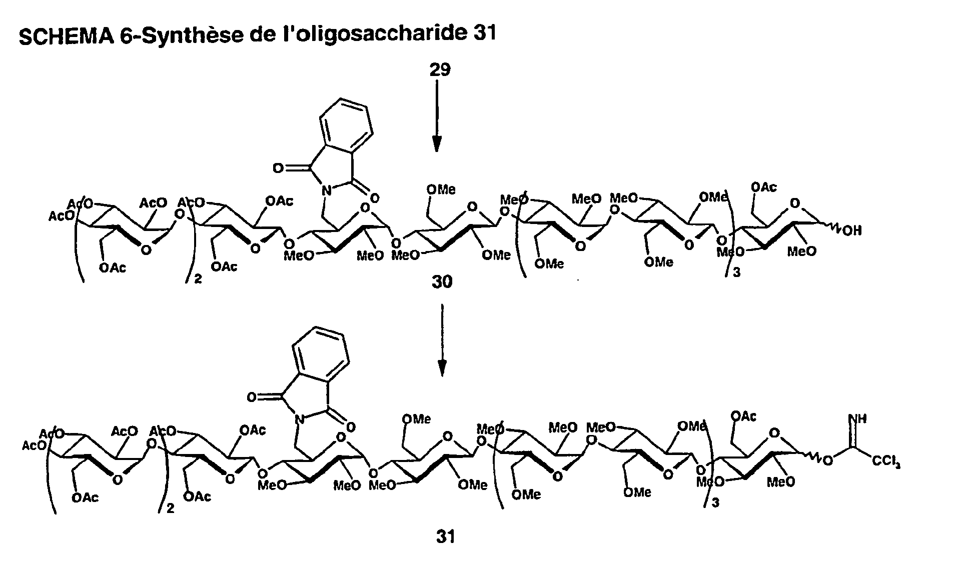

### PREPARATION 27

### (2,3,4,6-Tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-désoxy 2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-(2,3,6-triO-méthyl-β-D-glucopyranosyl)-(1→4)]₃-6-O-acétyl-2,3-di-O-méthy-D-glucopyranose (30)

On agite pendant 1 heure une solution du composé 29 (579 mg, 0,192 mmol) dans un mélange d'acide trifluoroacétique/dichlorométhane 2/1 (v/v) (11,5 mL). On dilue avec un mélange toluène/acétate de *n*-propyl 2/1 (v/v) (69 mL), on concentre et on co-évapore avec du toluène. On purifie le résidu par chromatographie sur colonne de gel de silice (toluène/éthanol 5/1 (v/v)) pour obtenir 523 mg de du composé 30.
CCM : Rf = 0,31, gel de silice, toluène/éthanol 5/1 (v/v)

### PREPARATION 28

### (2,3,4,6-Tétra-O-acétyl-α-D-glucopyranosyl)-(1→4)-(2,3,6-tri-O-acétyl-α-D-glucopyranosyl)-(1→4)-(2,3, 6-tri-O-acétyl-β-D-glucopyranosyl)-(1→4)-(6-désoxy-2,3-di-O-méthyl-6-phthalimido-α-D-glucopyranosyl)-(1→4)-(2,3,6-tri-O-méthyl-β-glucopyranosyl)-(1→4)-[(2,3,6-tri-O-méthyl-α-D-glucopyranosy)-(1→ 4)-(2,3,6-tri-O-méthyl-β-D-glucopyranosyl)-(1→4)]₃-6-O-acétyl-2,3-di-O-méthyl-D-glucopyranose trichloroacétimidate (31)

Du trichloroacétonitrile (65,5 µL, 0,85 mmol) et du carbonate de césium (88,4 mg, 0,27 mmol) sont ajoutés à une solution du composé 30 dans le dichlorométhane (3 mL). Après agitation pendant 2 heures, le mélange est filtré, concentré. Le résidu est purifié par chromatographie sur colonne de gel de silice (toluène/éthanol 6/1 (v/v) + 0,1% de triéthylamine) pour donner 477 mg du composé 31.
CCM : Rf = 0,35, gel de silice, toluène/éthanol 6/1 (v/v)

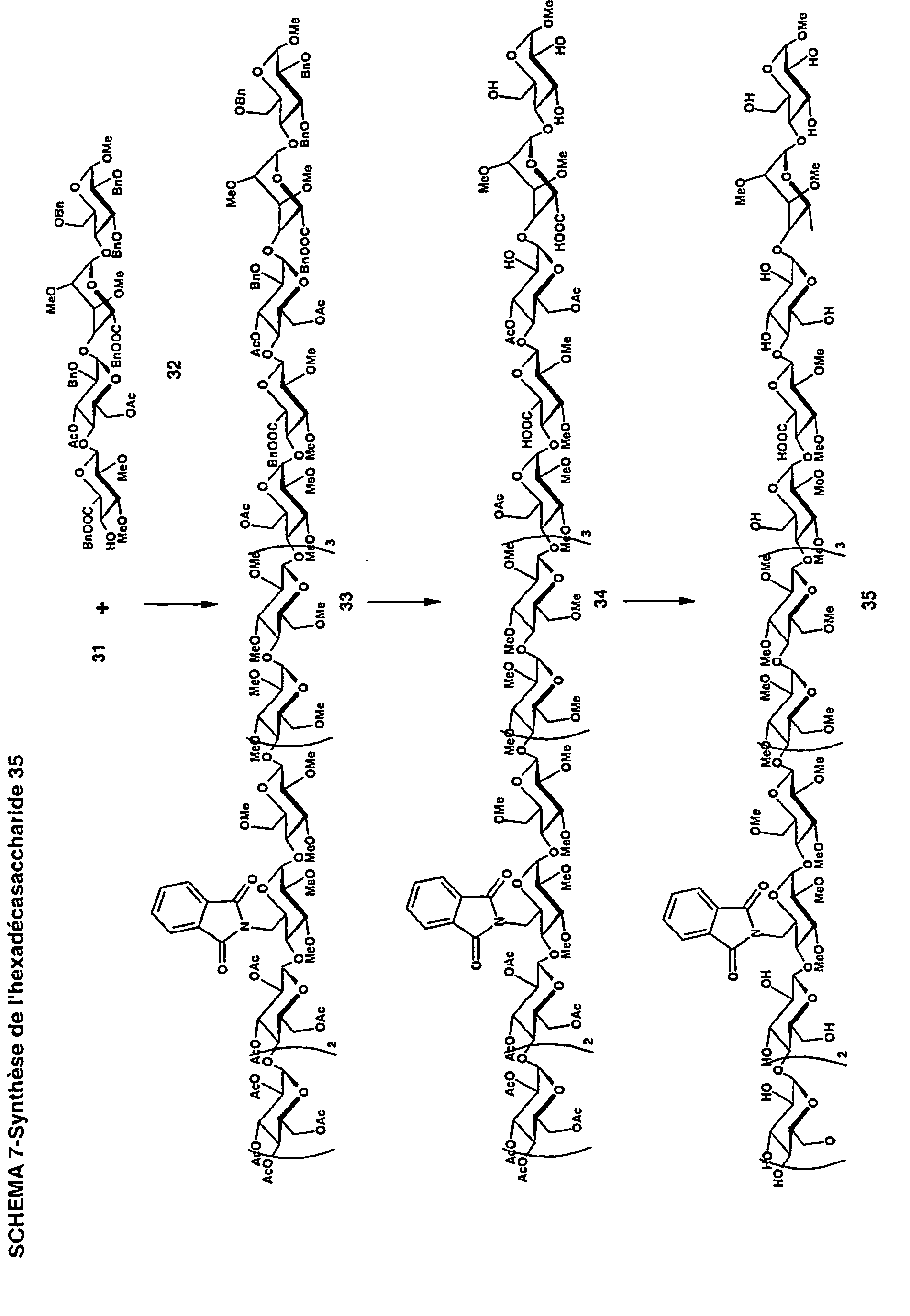

### PREPARATION 29

### Méthyl (2,3,4,6-tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-désoxy-2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-acétyl-2,3-di-O-méthyl-α-D-glucopyranosyl)-(1→4)-(benzyl 2,3-di-O-méthyl-β-D-glucopyranosyluronate)-(1→4)-(3,6-di-O-acétyl-2-O-benzyl-α-D-glucopyranosyl)-(1→4)-(benzyl 2,3.di-O-méthyl-α-L-idopyranosyluronate)- (1→4)-2,3,6-tri-O-benzyl-α-D-glucopyranoside (33)

On dissout l'imidate 31 (370 mg, 0,121 mmol) et le composé 32 (336 mg, 0,242 mmol), (obtenu selon P. Westerduin, et al. BioOrg. Med. Chem, 1994, 2, 1267) dans un mélange dichlorométhane/diéthyléther 1/2 (v/v) (5,5 mL). Après addition de tamis moléculaire 4Å en poudre, le mélange est refroidi à - 20 °C et on ajoute une solution 0,1 M de trifluorométhanesulfonate de triméthylsilyle dans le dichlorométhane (181,5 µL). Après 25 minutes, le mélange est neutralisé par addition d'hydrogénocarbonate de sodium solide. Après filtration et concentration, le résidu est purifié par chromatographie sur gel Sephadex® LH20, suivi par une chromatographie sur colonne de gel de silice (toluène/acétone 6/5 (v/v)) pour donner 302 mg du composé 33.
[α]_{D} = +86° (c =1, dichlorométhane).

### PREPARATION 30

### Méthyl (2,3,4,6-tétra-O-acétyl-α-D-glucopyranosyl)-(1→4)-(2,3,6-tri-O-acétyl-α-D-glucopyranosyl)-(1→4)-(2,3,6-tri-O-acétyl-β-D-glucopyranosyl)-(1→4)-(6-désoxy 2,3-di-O-méthyl-6-phthalimido-α-D-glucopyranosyl)-(1→4)-(2,3,6-tri-O-méthy-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-acétyl-2,3-di-O-méthyl-α-D-glucopyranosyl)-(1→4)-(acide 2,3-di-O-méthyl-β-D-glucopyranosyluronique)-(1→4)-(3,6-di-O-acétyl-α-D-glucopyranosyl)-(1→4)-(acide 2,3-di-O-méthyl-α-L-idopyranosyluronique)- (1→4)-α-D-glucopyranoside (34)

Une solution du composé 33 (104 mg, 0,024 mmol) dans l'acide acétique (5 mL) est traitée sous pression d'hydrogène (4 bars) en présence de palladium sur charbon 10% (104 mg) pendant 4 heures. Après filtration, la solution est lyophilisée pour donner le composé 34 (87 mg) qui est utilisé dans l'étape suivante sans purification.

### PREPARATION 31

### Méthyl (α-D-glucopyranosyl)-(1→4)-(α-D-glucopyranosyl)-(1→4)-(β-D-glucopyranosyl)-(1→4)-(6-désoxy-2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2,3-di-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)-(1→4)-α-D-glucopyranoside (35)

A une solution du composé 34 (80 mg, 0,021 mmol) dans le méthanol anhydre (6,9 mL) en présence de tamis moléculaire 3Å en poudre (875 mg), on ajoute une solution molaire de méthylate de sodium dans le méthanol (140 µL). Après 20 heures à température ambiante, on filtre et on neutralise avec de l'acide acétique. La solution est concentrée de moitié et déposée sur colonne de Sephadex® G-25 fine (3 x 92 cm). Après élution par de l'eau et lyophilisation, on obtient le composé 35 (66 mg).

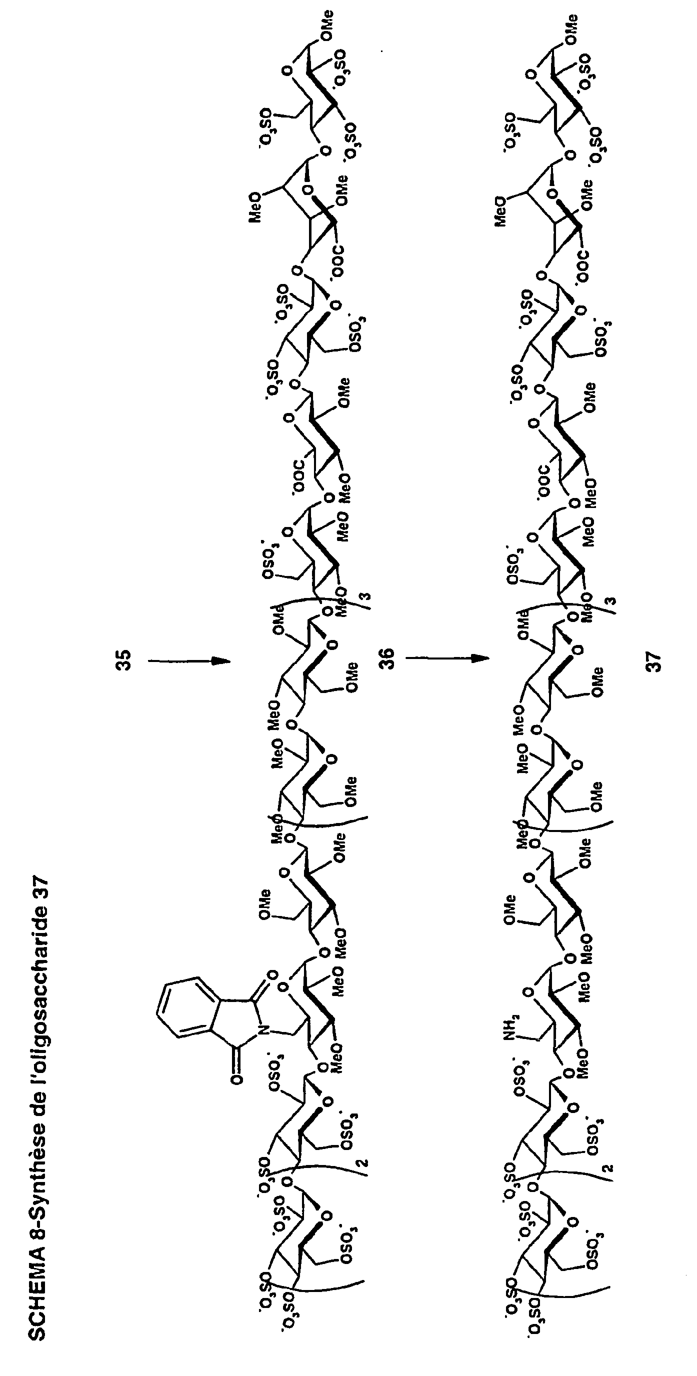

### PREPARATION 32

### Méthyl (2,3,4,6-tétra-O-sulfonat-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(6-désoxy 2,3-di-O-méthyl-6-phthalimido-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-sulfonato-2,3-di-O-méthyl-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (36)

Le polyol 35 (66,4 mg, 0,021 mmol) est dissous dans du *N,N*-diméthylformamide (1,8 mL). On ajoute le complexe trioxyde de sulfure-triéthylamine (320 mg, 1,77 mmol) et le mélange est agité pendant 20 heures à 55°C. On dépose la solution sur une colonne de Sephadex® G-25 fine (3 x 92 cm) éluée par du chlorure de sodium 0,2 M. On concentre les fractions contenant le produit et on dessale en utilisant la même colonne éluée par l'eau. Après lyophilisation, on obtient 83 mg du composé 36.
Masse : méthode "ESI", mode négatif : masse chimique = 4968,92 ; masse expérimentale = 4966,52 ± 0,16 u.m.a.

### PREPARATION 33

### Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(6-amino-6-désoxy-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-n-glucopyranosyl)-(1→4)-O-(2,3,6-tri-O-méthyl-β-D-glucopyranosyl)-(1→4)]₃-(6-O-sulfonato-2,3-di-O-méthyl-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (37)

On dissout le composé 36 (83 mg, 0,017 mmol) dans un mélange éthanol/eau 2/1 (v/v) (1,67 mL). On ajoute l'hydrate d'hydrazine (81,2 µL, 1,67 mmol) et le mélange est porté à reflux pendant 20 heures. On dépose la solution sur une colonne de Sephadex® G-25 fine (3 x 92 cm) éluée par l'eau. Après concentration des fractions contenant le produit, le résidu est dissous dans éthanol/eau 2/1 (v/v) (5,00 mL) et à nouveau traité comme dans les conditions précédentes par l'hydrate d'hydrazine (81,2 µL) pour donner 71 mg du composé 37.
Masse : méthode "ESI", mode négatif : masse chimique = 4838,32 ; masse expérimentale = 4814,6 ± 0,70 u.m.a.

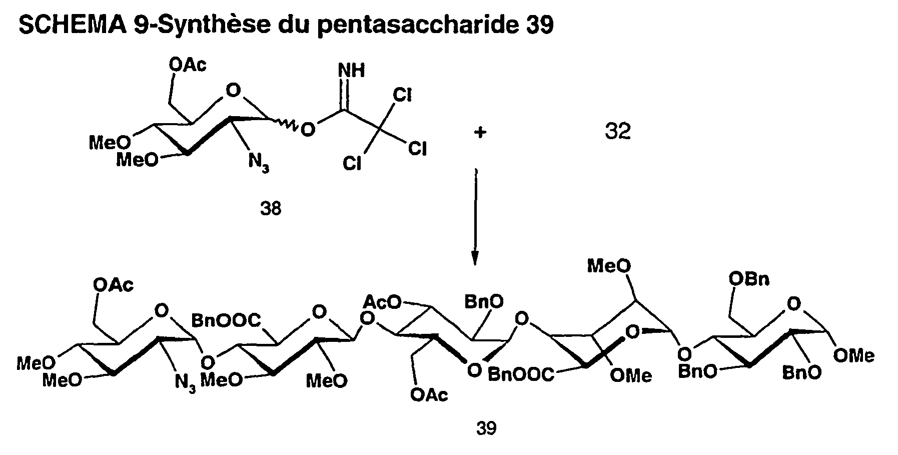

### PREPARATION 34

### Méthyl (6-O-acétyl-2-azido 2-désoxy-3,4-di-O-méthyl-α-D-glucopyranosyl)-(1→4)-(benzyl 2,3-di-O-méthyl-β-D-glucopyranosyluronate)-(1-4)-(3,6-di-O-acéiyl-2-O-benzyl-α-D-glucopyranosyl)-(1→4)-(benzyl 2,3-di-O-méthyl-α-L-idopyranosyluronate)-(1→4)-2,3,6-tri-O-benzyl-α-D-glucopyranoside (39)

On dissout le composé *6-*O*-acétyl-2-azido-2-désoxy-3,4-di-O-méthyl-α,β-*D-*glucopyranose trichloroacétimidate* 38 (265 mg, 0,631 mmol) (obtenu selon J. Basten, et al. Bioorg. Med. Chem. Lett. (1992), 2(9), 901) et le composé 32 (584 mg, 0,420 mmol) (obtenu selon P. Westerduin, et al. BioOrg. Med. Chem, 1994, 2, 1267) dans un mélange dichlorométhane/diéthyléther 1/2 (v/v) (28,5 mL). Après addition de tamis moléculaire 4Å en poudre, le mélange est refroidi à -20°C et on ajoute une solution 0,1 M de trifluorométhanesulfonate de triméthylsilyle dans le dichlorométhane (94,6 µL). Après 10 minutes, on ajoute à nouveau de l'imidate (53,8 mg) puis une solution 0,1 M de trifluorométhanesulfonate de triméthylsilyle dans le dichlorométhane (19,2 µL). Après 10 minutes, le mélange est neutralisé par addition d'hydrogénocarbonate de sodium solide. Après filtration et concentration, le résidu est purifié par chromatographie sur colonne de gel de silice (toluène/acétate d'éthyle 3/1 (v/v)) pour donner 499 mg du composé 39.
[α]_{D} = +66° (c =1,07, dichlorométhane).

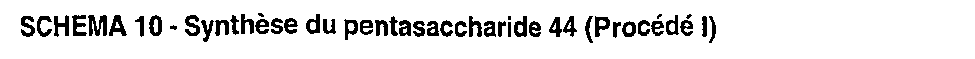

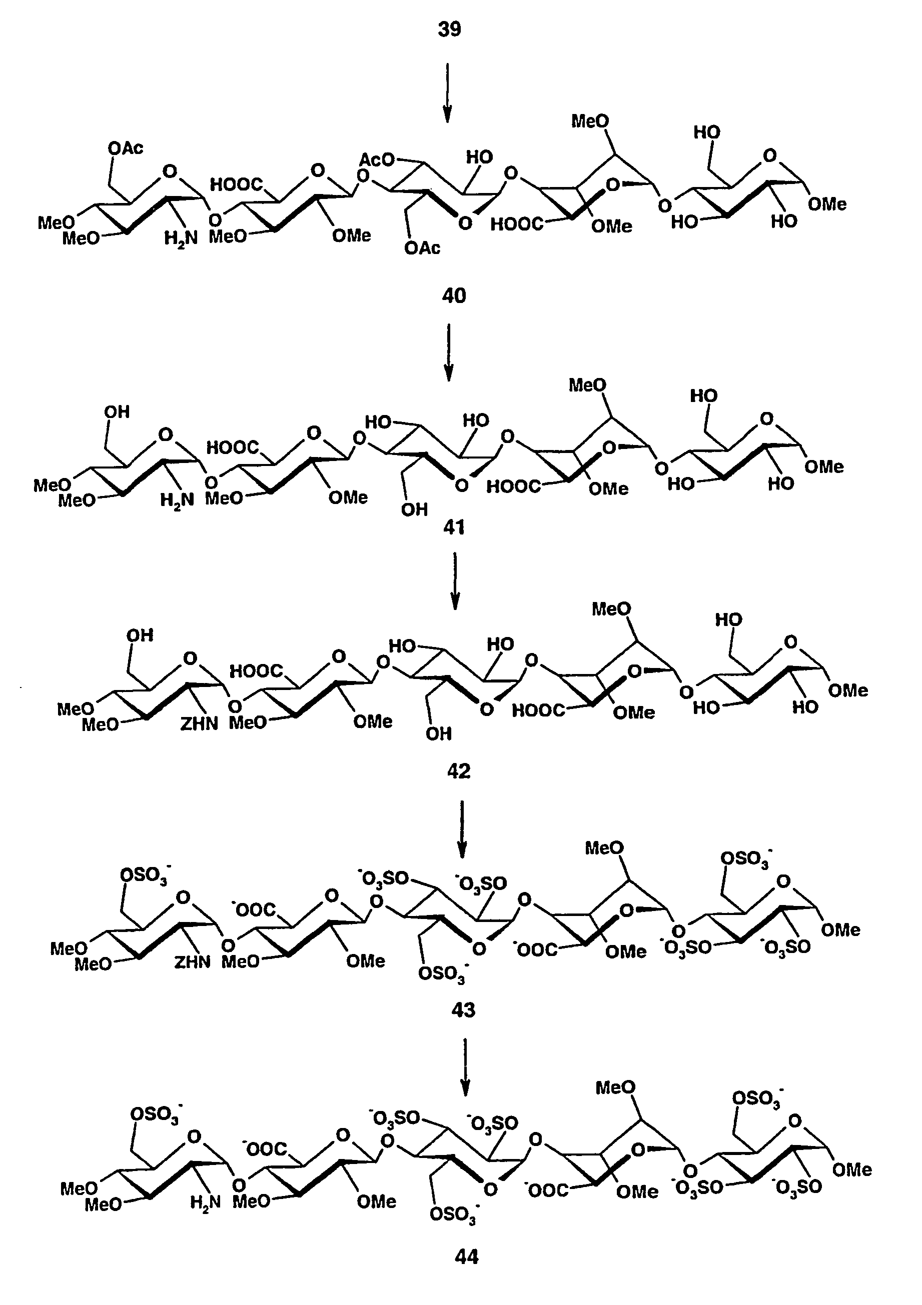

### PREPARATION 35

### Méthyl (6-O-acétyl-2-amino-2-désoxy-3,4-di-O-méthyl-α-D-glucopyranosyl)-(1→4)-(acide 2,3-di-O-méthyl-β-D-glucopyranosyluronique)-(1→4)-(3,6-di-O-acétyl-α-D-glucopyranosyl)-(1→4)-(acide 2,3-di-O-méthyl-α-L-idopyranosyl-uronique)-(1→4)-α-D-glucopyranoside (40)

Une solution du composé 39 (552,6 mg, 0,335 mmol) dans un mélange *tert*-butanol/acétate d'éthyle 5/1 (v/v) (16 mL) est traitée sous pression d'hydrogène (10 bars) en présence de palladium sur charbon 10% (1,10 g) et d'acide chlorhydrique 1 M (0,336 mL) pendant 4 heures 30. Après filtration, la solution est concentrée et donne le composé 40 qui est utilisé dans l'étape suivante sans purification.

### PREPARATION 36

### Méthyl (2-amino-2-désoxy-3,4-di-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)-(1→4)-α-D-glucopyranoside (41)

On dissout le composé 40 (324 mg, 0,300 mmol) dans du méthanol (8,8 mL). On ajoute une solution 5 M d'hydroxyde de sodium (2,2 mL) et on agite à température ambiante pendant 16 heures. On neutralise avec une résine Dowex^{®} 50 H⁺ et on filtre. On passe la solution au travers d'une colonne de Sephadex® G-25 fine éluée par l'eau. On concentre les fractions contenant le produit et on obtient 254,2 mg du composé 41. A ce stade on vérifie par RMN que les groupements protecteurs (benzyles et acétyles) ont été enlevés.
CCM : Rf = 0,26, gel de silice, acétate d'éthyle/pyridine/acide acétique/eau 5/5/1/3 (v/v/v/v)

### PREPARATION 37

### Méthyl (2-(benzyloxycarbonyl)amino-2-désoxy-3,4-di-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)-(1→4)-α-D-glucopyranoside (42)

On dissout le composé 41 (241,1 mg, 0,253 mmol) dans l'eau (12,4 mL) et on ajoute de l'hydrogénocarbonate de sodium (63,7 mg) puis goutte à goutte du chloroformiate de benzyle (41 µL). Après 12 heures d'agitation, on passe le mélange réactionnel au travers d'une colonne de Sephadex® G-25 fine éluée par l'eau. On concentre les fractions contenant le produit. La purification par chromatographie sur colonne de gel de silice (acétate d'éthyle/pyridine/acide acétique/eau 21/17/3,6/10 v/v/v/v) donne 221 mg du composé 42.
CCM : Rf = 0,63, gel de silice, acétate d'éthyle/pyridine/acide acétique/eau 5/5/1/3 (v/v/v/v)

### PREPARATION 38

### Méthyl (2-(benzyloxycarbonyl)amino-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (43)

Le polyol 42 (19,6 mg, 0,018 mmol) est dissous dans du *N,N* diméthylformamide (1,62 mL). On ajoute le complexe trioxyde de sulfure-triéthylamine (114 mg) et le mélange est agité pendant 20 heures à 55°C à l'abri de la lumière. On dépose la solution sur une colonne de Sephadex® G-25 fine éluée par du chlorure de sodium 0,2 M. On concentre les fractions contenant le produit et on dessale en utilisant la même colonne éluée par l'eau. Après lyophilisation, on obtient 28,5 mg du composé 43.
[α]_{D} = +48° (c = 2,75, eau).

### PREPARATION 39

### Méthyl (2-amino-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium (44)

Une solution du composé 43 (27,5 mg, 0,015 mmol) dans un mélange *tert*-butanol (333 µL)-eau (500 µL) est traitée sous pression d'hydrogène ( 5 bars) en présence de palladium sur charbon 10% (8,25 mg) pendant 16 heures. Après filtration, la solution est concentrée et le résidu est déposé sur colonne de Sephadex® G-25 fine (3 x 92 cm). Après élution par l'eau et lyophilisation, on obtient 23,7 mg du composé 44.
[α]_{D} = +58° (c = 1, eau).

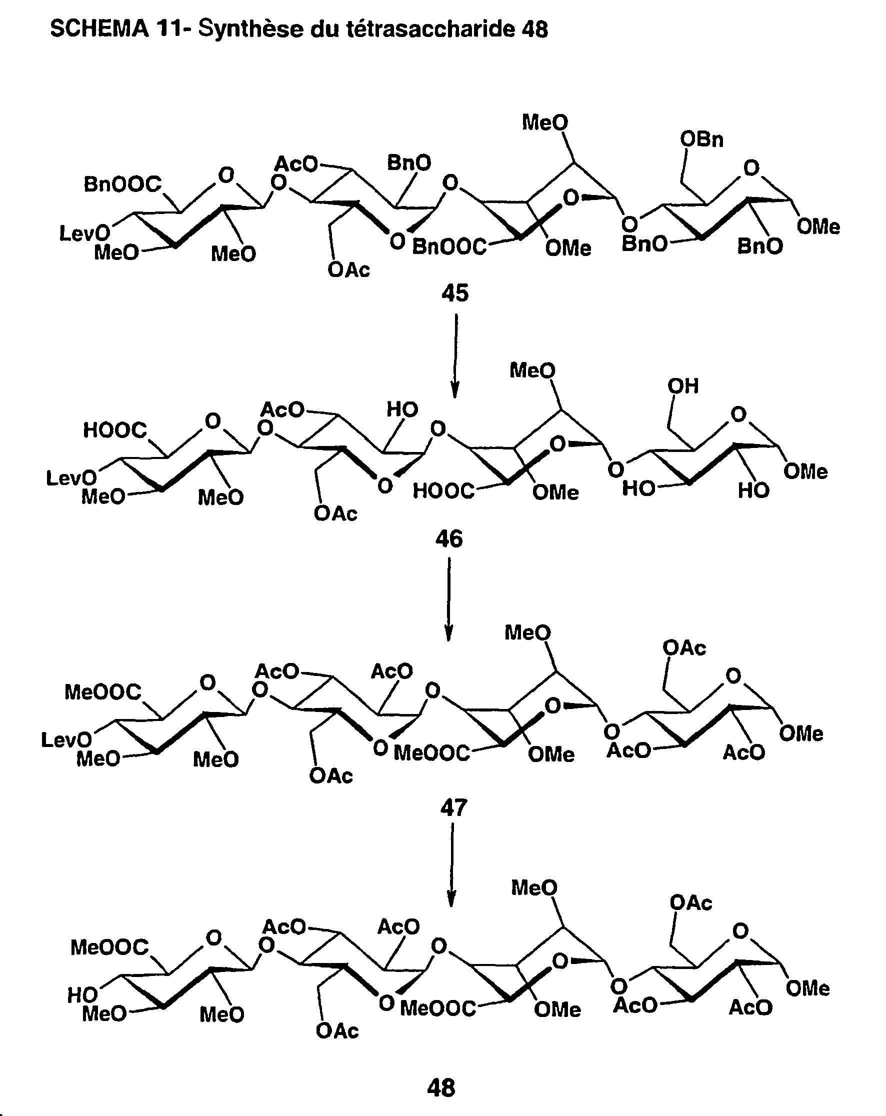

### PREPARATION 40

### Méthyl (acide 4-O-lévulinyl-2,3-di-O-méthyl-β-D-glucopyranosyluronique)-(1→4)-(3,6-di-O-acétyl-α-D-glucopyranosyl)-(1→4)-(acide 2,3-di-O-méthyl-α-L-idopyranosyluronic)-(1→4)-α-D-glucopyranoside (46)

Une solution du composé 45 (4,50 g, 3,02 mmol) (obtenu selon P. Westerduin, et al. BioOrg. Med. Chem. 1994, 2, 1267) dans un mélange acétate d'éthyle/*tert*-butanol (72 mL, 1/1, v/v) est traitée sous pression d'hydrogène (4 bars) en présence de palladium sur charbon 10% (9,0 g) pendant 6 heures. Après filtration et concentration, le composé 46 obtenu est directement engagé dans l'étape suivante sans purification.
CCM : Rf = 0,54, acétate d'éthyle/pyridine/acide acétique/eau 26/22/4,6/17 v/v/v/v.

### PREPARATION 41

### Méthyl (méthyl 4-O-lévulinyl-2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α -L-ido-pyranosyl-uronate)-(1→4)-2,3,6-tri-O-acétyl-α-D-glucopyranoside (47)

A une solution du composé 46 (2,57 g, 2,71 mmol) dans du *N,N-*diméthylformamide anhydre (35 mL) on ajoute, à 0 °C, de l'hydrogénocarbonate de potassium (2,71 g) puis de l'iodure de méthyle (3,4 mL). Après 16 h d'agitation à température ambiante, le milieu réactionnel est refroidi à 0°C. On ajoute alors successivement de la diméthylaminopyridine (132 mg) puis de l'anhydride acétique 1,5 mL). Le mélange est agité pendant 16 h. Après neutralisation de l'excès d'anhydride acétique, on dilue avec de l'acétate d'éthyle. La phase organique est lavée successivement avec une solution d'hydrogénosulfate de potassium à 10% et de l'eau puis avec une solution d'hydrogénocarbonate de sodium saturée et de l'eau, séchée sur sulfate de sodium, filtrée puis évaporée à sec. Le résidu est purifié par chromatographie sur colonne de gel de silice [cyclohexane/(acétate d'éthyle/éthanol 1/1) 9/5] pour donner 2,51 g du composé 47.
CCM : Rf = 0,41, toulène/acétone 2/1 v/v.

### PREPARATION 42

### Méthyl (méthyl 2,3-di-O-méthyf-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,3,6-tri-O-acétyl-α-D-glucopyranoside (48)

Le composé 47 (2,5 g, 2,56 mmol) est dissous dans un mélange toluène/éthanol 1/1 v/v (500 mL). On ajoute de l'acétate d'hydrazine (1,01 g). Après 2h d'agitation à température ambiante, le milieu réactionnel est concentré à sec. Le résidu est dissous dans le dichlorométhane. La phase organique est lavée successivement avec une solution d'hydrogénocarbonate de sodium à 2% et de l'eau, séchée sur sulfate de sodium, filtrée puis évaporée à sec. Après chromatographie sur colonne de gel de silice (toluène/acétate d'éthyl 1/4 v/v), on obtient 2,01 g du composé 48.
CCM : Rf = 0,37, toluène/acétone 2/1 v/v.

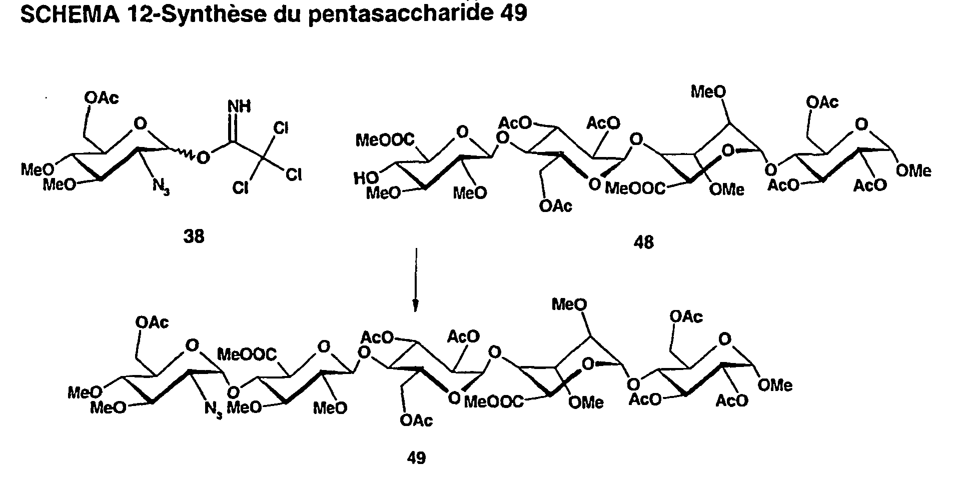

### Préparation 43

### Méthyl (6-O-acétyl-2-azido-2-deoxy-3,4-di-O-méthyl-α-D-glucopyranosyl)-(1→4)-(méthyl 2,3-di-O-méthyl-β-D-glucopyranosyluronate)-(1→4)-(2,3,6-tri-O-acétyl-α-D-glucopyranosyl)-(1→4)-(méthyl 2,3-di-O-méthyl-α-L-idopyranosyluronate)-(1→4)-2,3,6-tri-O-acétyl-α-O-glucopyranoside (49)

On dissous l'imidate 38 (1,18 g, 2,81 mmol) (obtenu selon J. Basten et al. Bioorg. Med. Chem. Lett. (1992), 2(9), 901) et le composé 48 (1,83 g, 1,75 mmol) dans un mélange dichlorométhane/diéthyléther 1/2 (v/v) (126 mL). Après addition de tamis moléculaire 4Å en poudre, le mélange est refroidi à - 20 °C et on ajoute une solution 1 M de trifluoro-méthanesulfonate de *tert*-butyldiméthylsilyle dans le dichlorométhane (421 µL). Après 30 minutes, on ajoute une nouvelle quantité d'imidate (266 mg) ainsi qu'une solution 1 M de trifluorométhanesulfonate de tert-butyldiméthylsilyle dans le dichlorométhane (168 µL). Après 10 minutes, le mélange est neutralisé par addition d'hydrogénocarbonate de sodium solide et on filtre. On reprend avec du dichlorométhane, on lave successivement avec une solution d'hydrogénocarbonate de sodium à 2% et de l'eau, séché sur sulfate de sodium puis concentré sous vide. Le résidu obtenu est purifié par une chromatographie sur colonne de gel de silice (dichlorométhane/acétate d'éthyle 4/3 puis 1/1 v/v) pour donner 1,814 g du composé 49.
CCM : Rf = 0,57, toluène/acétate d'éthyle 3/1 v/v.
[α]_{D} = +93° (c =1,15, dichlorométhane).

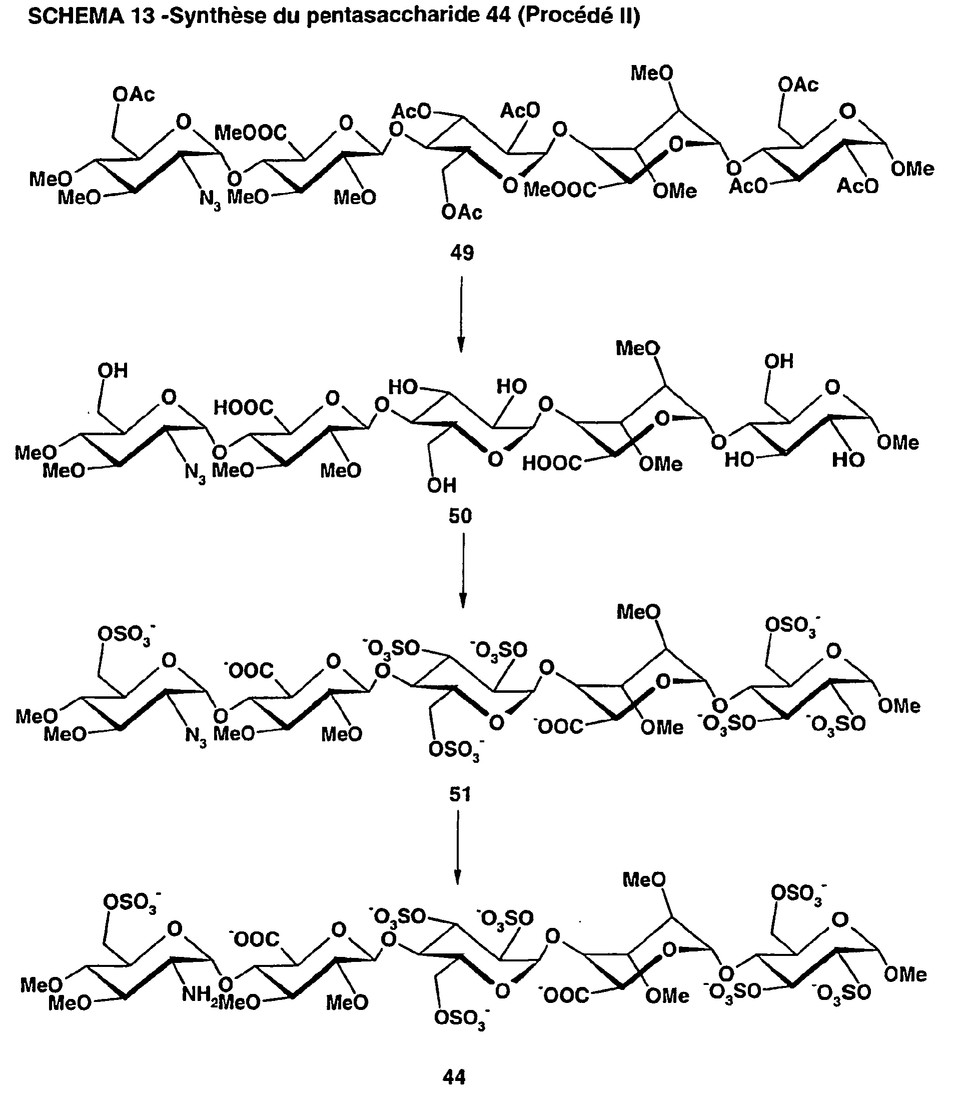

### PREPARATION 44

### Méthyl (2-azido-2-deoxy-3,4-di-O-méthyl-α-D-glucopyranosyl)-(1→4)-(acide 2,3-di-O-méthyl-β-D-glucopyranosyluronique)-(1→4)-(α-D-glucopyranosyl)-(1→4)-(acide 2,3-di-O-méthyl-α-L-idopyranosyluronique)-(1→4)-α-D-glucopyranoside (50)

De l'eau oxygénée à 30% (42 mL) est ajoutée, à - 5 °C, à une solution du composé 49 (845,3 mg) dans le tétrahydrofurane (104 mL). Après 5 minutes d'agitation, on ajoute goutte à goutte une solution aqueuse 0,7 M d'hydroxyde de lithium (19,2 mL).

Le mélange réactionnel est agité pendant 1 h à - 5 °C, puis pendant 4 h à 0 °C et finalement pendant 16 h à température ambiante. On neutralise avec une solution 1 M d'acide chlorhydrique.

On dépose la solution sur une colonne de Sephadex® G-25 fine (5 x 1000 cm) éluée par de l'eau. Les fractions contenant le composé attendu sont réunies, concentrées et déposées sur une colonne de résine Dowex AG 50 WX4 H⁺ (50 mL). On recueille le composé à 0 °C et on concentre pour obtenir 618 mg du composé 50.
CCM : Rf = 0,56, acétate d'éthyle/pyridine/acide acétique/eau 26/22/4,6/17 v/v/v/v.

### PREPARATION 45

### Méthyl (2-azido-2-désoxy-3,4-di-O-méthyl-6-O-sulfonato-α-D-glucopyranosyl)-(1→4)-(2,3-di-O-méthyl-β-D-glucopyranosyluronate)-(1→4)-(2,3,6-tri-O-sulfonato-α-D-gluco-pyranosyl)-(1→4)-(2,3-di-O-méthyl-α-L-idopyranosyluronate)-(1→4)-2,3,6-tri-O-sulfonato-α-D-glucopyranoside, sel de sodium (51)

Juste avant de l'utiliser, le composé 50 est co-distillé avec du *N,N*-diméthylformamide (3 x 29 mL). A une solution du composé 50 (612 mg, 0,624 mmol) dans le *N*,*N*-diméthylformamide (58 mL), on ajoute le complexe trioxyde de sulfure-triéthylamine (3,84 g).

Le mélange est agité pendant 16 heures à 55°C à l'abri de la lumière. Le mélange refroidi à 0 °C est ajouté goutte à goutte à une solution d'hydrogénocarbonate de sodium (5,33 g) dans l'eau (200 mL). On agite pendant 16 h à température ambiante et on concentre à sec.

Le résidu est dissous dans l'eau et on dépose la solution sur une colonne de Sephadex® G-25 fine éluée par du chlorure de sodium 0,2 M. On concentre les fractions contenant le produit et on dessale en utilisant la même colonne éluée par l'eau. Après lyophilisation, on obtient 1,06 g du composé 51.
CCM : Rf = 0,5, acétate d'éthyle/pyridine/acide acétique/eau 3/5/1/3 v/v/v/v.

### PREPARATION 46

### Méthyl (2-amino 2-désoxy 3,4-di-O-méthyl-6-O-sulfonato-α-_{D}-glucopyranosyl)-(1→4)-(acide 2,3-di-O-méthyl-β-_{D}-glucopyranosyluronique)-(1→4)-(2,3,6-tri-O-suifonato-α-D-gluco-pyranosyl)-(1→4)-(acide 2,3-di-O-méthyl-α-L-idopyranosyluronique)-(1→4)- 2,3,6-tri-O-sulfo-nato-α-_{D}-glucopyranoside, sel de sodium (44)

Cette hydrogénolyse a été réalisée en deux fois et à chaque fois sur 534,4 mg du composé 51.

Une solution du composé 51 (534,4 mg) dans un mélange *tert*-butanol (6,7 mL, 12,6 mL/g)-eau (10 mL, 19 mL/g) est traitée sous pression d'hydrogène (5 bars) en présence de palladium sur charbon 10% (160 mg) à 40 °C pendant 4 heures. Après filtration ( filtre Millipore^{®} LSWP 5 µm), la solution est concentrée à sec pour donner 530 mg du composé 44.
CCM : Rf = 0,49, acétate d'éthyle/pyridine/acide acétique/eau 3/5/1/3 v/v/v/v.

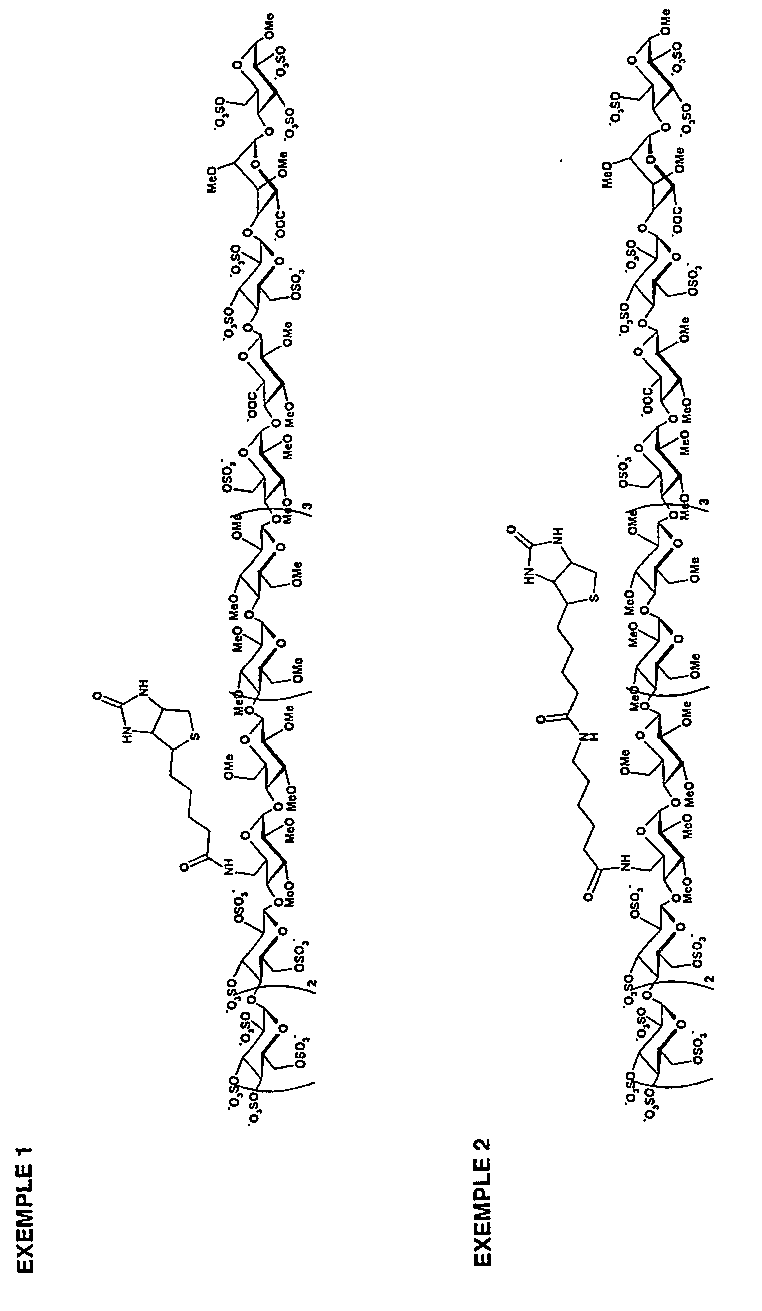

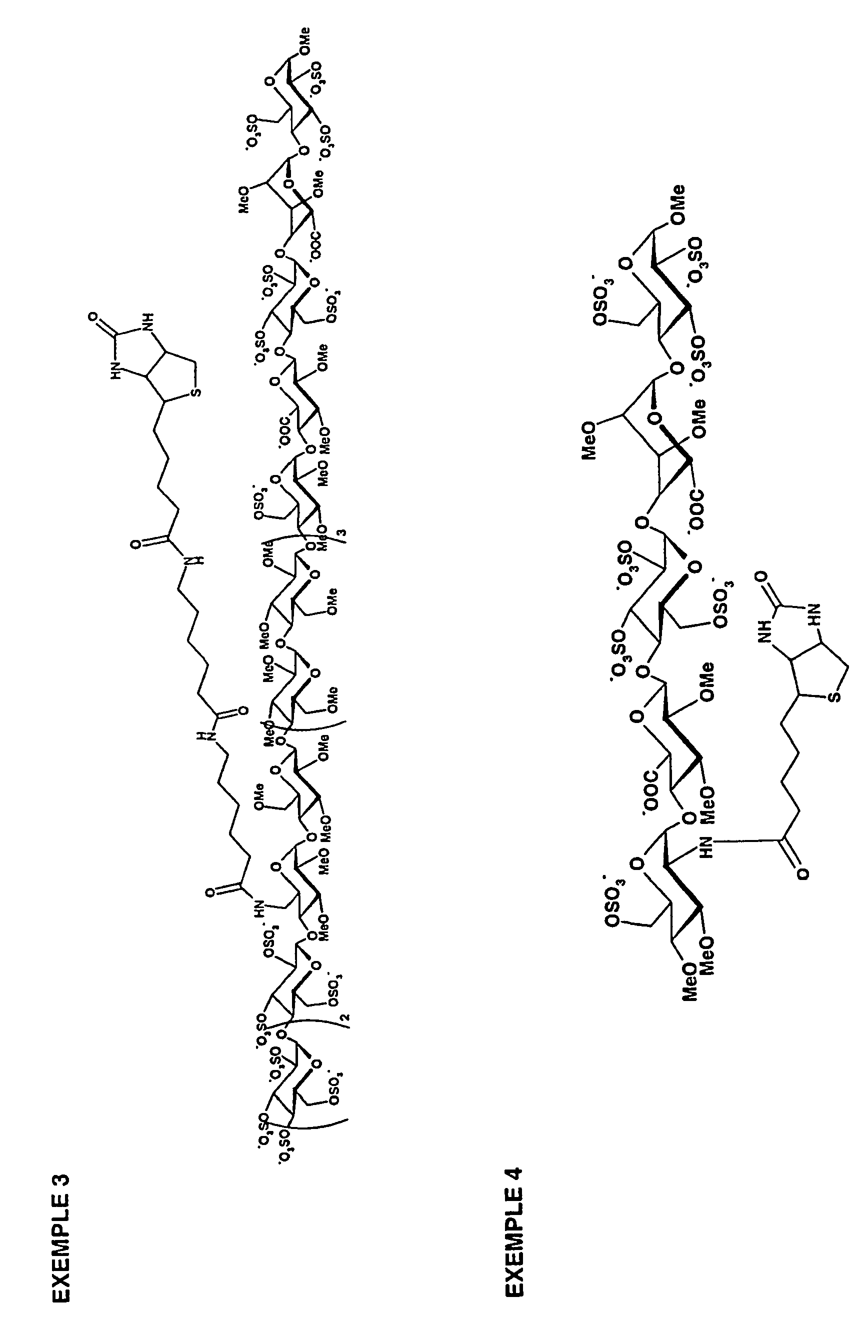

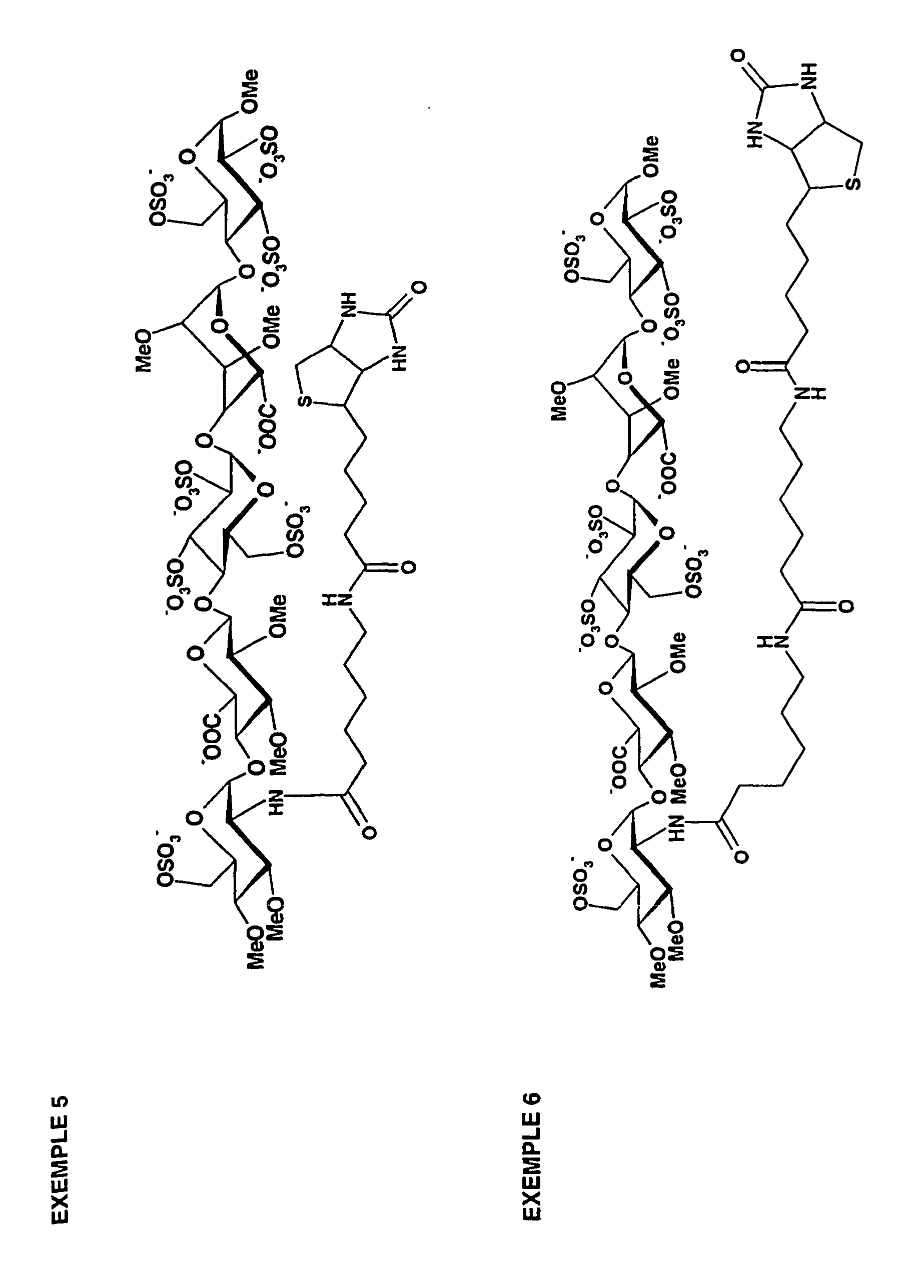

### EXEMPLE 1

### Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(6-biotinamido-6-désoxy-2,3-di-O-méthyl-α-D-glucopyranosyl)-(1→4)-(2,3,6-tri-O-méthyl-β-D-glucopyranosy)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-sulfonato-2,3-di-O-méthyl-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

A une solution du composé 37 (18 mg, 3,72 µmol) dans de l'hydrogénocarbonate de sodium à 0,5% (1,5 mL), on ajoute du sulfosuccinimide de biotine (16,5 mg).

Après 16 heures d'agitation à température ambiante, on dépose le mélange réactionnel sur colonne de Sephade® G-25 fine éluée par du chlorure de sodium.

On concentre les fractions contenant le produit et on dessale sur la même colonne éluée par de l'eau.
Après lyophilisation, on obtient 15,9 mg du composé de l'EXEMPLE 1.
[α]_{D} = +59° (c = 0,78, eau).
Masse : méthode "ESI", mode négatif : masse chimique = 5065,12 ; masse expérimentale = 5064,18 ± 1,04 u.m.a.

### EXEMPLE 2

### Méthyl (2,3,4,6-tétra-O-sulfonato-α-D-glucopyranosyl)-(1→4)-(2,3, 6-tri-O-sulfonato-α-D-glucopyranosyl)-(1→4)-(2,3,6-tri-O-sulfonato-β-D-glucopyranosyl)-(1→4)-(6-[6-(biotinamido) hexamido]-6-désoxy-2,3-di-O-méthyl-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(6-O-sulfonato-2,3-di-O-méthyl-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

A une solution du composé 37 (18 mg, 3,72 µmol) dans de l'hydrogénocarbonate de sodium à 0,5% (1,5 mL), on ajoute du 6-(biotinamido) hexanoate de (16,5 mg). Après 16 heures d'agitation à température ambiante, on dépose le mélange réactionnel sur colonne de Sephadex® G-25 fine éluée par du chlorure de sodium.

On concentre les fractions contenant le produit et on dessale sur la même colonne éluée par de l'eau.

Après lyophilisation, on obtient 17,9 mg du composé de l'EXEMPLE 2.
[α]_{D} = +60° (c =1,0, eau).
Masse : méthode "ESI", mode négatif : masse chimique = 5178,28 ; masse expérimentale = 5176,3 ± 0,77 u.m.a.

### EXEMPLE 3

### Méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(6 [6-(6-biotanamidohexamido) hexamido]-6-désoxy-2,3-di-O-méthyl-α-D-glucopyranosyl)-(1→4)-(2,3,6-tri-O-méthy-β-D-glucopyranosyl)-(1→4)-[(2,3,6-triO-méthyl-α-D-glucopyranosyl)-(1→4)-0-(2,3,6-tri-O-méthy-β-D-glucopyranosyl)-(1→4)]₃-(6-O-sulfonato-2,3-di-O-méthyl-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

A une solution du composé 37 (17 mg, 3,51 µmol) dans de l'hydrogénocarbonate de sodium à 0,5% (1,4 mL), on ajoute du 6-(6-biotinamidohaxamido) hexanoate de sulfosuccinimidyle (23,6 mg).

Après 16 heures d'agitation à température ambiante, on dépose le mélange réactionnel sur colonne de Sephadex® G-25 fine éluée par du chlorure de sodium. On concentre les fractions contenant le produit et on dessale sur la même colonne éluée par de l'eau.

Après lyophilisation, on obtient 17,4 mg du composé de l'EXEMPLE 3.
[α]_{D} = +64° (c = 1,0, eau).
Masse : méthode "ESI", mode négatif : masse chimique = 5291,44 ; masse expérimentale = 5292,1 ± 0,83 u.m.a.

### EXEMPLE 4

### Méthyl (2-biotinamido-2-désoxy-3,4-di-O-méthyl-6-O-sulfonato-α-D-glucopyranosyl)-(1→4)-(acide 2,3-di-O-méthyl-β-O-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside, sel de sodium

A une solution du composé 44 (21,2 mg, 0,012mmol) dans de l'hydrogénocarbonate de sodium à 0.5% (750 µL), on ajoute une solution de *N*-hydroxysuccinimide de biotine (42 mg) dans du *N*,*N*-diméthylformamide (750 µL). Après 16 heures d'agitation à température ambiante, on dépose le mélange réactionnel sur colonne de Sephadex® G-25 fine.

Après élution par l'eau et lyophilisation, on obtient 22,3 mg du composé de l'EXEMPLE 4.
[α]_{D} = +38° (c = 0,15, eau).
Masse : méthode "ESI", mode négatif : masse chimique = 1938,48 ; masse expérimentale = 1937,48 ± 0,11 u.m.a.

### EXEMPLE 5

### Méthyl (2-[N-(6-biotinamido hexanoyl)]-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)- 2,3,6-tri-O-sulfonato-α-D-glucopyranoside, sel de sodium

Cette réaction a été réalisée en deux fois et à chaque fois sur 494,5 mg du composé 44.

Le composé 44 (494,5 mg, 0,289 mmol) est dissous dans une solution aqueuse d'hydrogénocarbonate de sodium à 0,5% (116 mL).

On y ajoute goutte à goutte une solution de 6-(biotinamido) hexanoate de sulfosuccinimide (1,46 g, 2,63 mmol) dans une solution d'hydrogénocarbonate de sodium à 0,5% (12 mL) Après 16 heures d'agitation à température ambiante, on ajoute une solution aqueuse d'hydroxyde de sodium 1 M et on agite pendant 1 h. On dépose le mélange réactionnel sur une colonne de Sephadex® G-25 fine(5 x 1000 cm) éluée par du chlorure de sodium.

Les fractions contenant le produit et provenant des deux réactions sont réunies.

Après lyophilisation, on obtient 999,2 mg de l'EXEMPLE 5.
CCM: Rf = 0,42, acétate d'éthyle/pyridine/acide acétique/eau 3/5/1/3 v/v/v/v.
Masse : méthode "ESI", mode négatif : masse chimique = 2051,64 ; masse expérimentale : 2051,60 ± 0,43 u.m.a.

### EXEMPLE 6

### Méthyl (2-[6-(6-biotinamidohexamido) hexamido]-2-désoxy-3,4-di-O-méthyl-6-O-suifonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-suifonato-α-D-glucopyranoside, sel de sodium.

Le composé 44 (30,1 mg, 17,6 µmol) est dissous dans une solution aqueuse d'hydrogéno carbonate de sodium à 0,5% (7 mL). On y ajoute goutte à goutte une solution de 6-(6-biotinamidohaxamido) de sulfosuccinimidyle (118 mg, 176 µmol) dans une solution d' hydrogénocarbonate de sodium à 0,5% (1 mL) Après 16 heures d'agitation à température ambiante, on ajoute une solution aqueuse d' hydroxyde de sodium 1 M et on agite pendant 1 h. On dépose le mélange réactionnel sur une colonne de Sephadex® G-25 fine(2 x 85 cm) éluée par du chlorure de sodium.

Les fractions contenant le produit sont réunies, concentrées et dessalées sur une colonne de Sephadex® G-25 fine(2 x 85 cm) éluée par de l'eau.

Après lyophilisation, on obtient 26,5 mg du composé de l'EXEMPLE 6.
Masse : méthode "ESI", mode négatif : masse chimique = 2164,48 ; masse expérimentale : 2164,29 ± 0,38 u.m.a.

## Revendications

1. Polysaccharides synthétiques à activité antithrombotique et leurs sels pharmaceutiquement acceptables **caractérisés en ce qu'**ils présentent au moins une liaison covalente avec la biotine ou un dérivé de la biotine.

2. Polysaccharides selon la revendication 1 de formule : 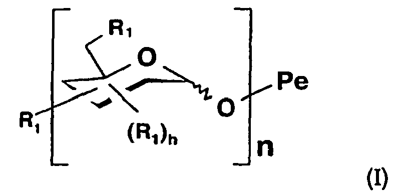 dans laquelle :
- le trait ondulé désigne une liaison située soit au-dessous soit au-dessus du plan du cycle pyranosique, 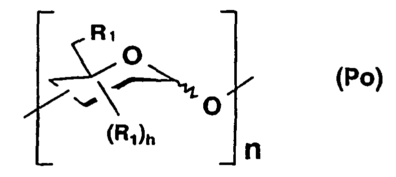 Po désigne un polysaccharide, contenant n unités monosaccharidiques identiques ou différentes, lié par son carbone anomère à Pe, 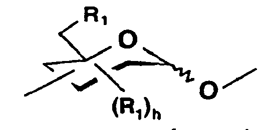 est une représentation schématique d'une unité monosaccharidique à structure pyranosique choisie parmi les hexoses, les pentoses et les sucres desoxy correspondants, cette unité étant liée par son carbone anomère à une autre unité monosaccharidique, et les groupes hydroxy de cette unité étant substitués par des groupes R₁ identiques ou différents, R₁ étant tel que défini ci-dessous,
- Pe représente un pentasaccharide de structure : 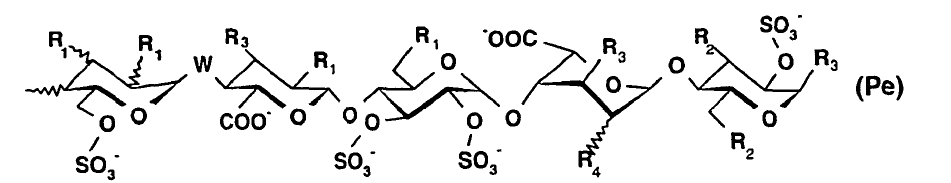
- h est égal à 1 ou 2,
- n est un entier et peut prendre toute valeur de 0 à 25,
- R₁ représente l'enchaînement -T-Biot, un groupe (C₁-C₆)alcoxy ou un groupe
- OSO₃⁻,
- R₂ représente l'enchaînement -T-Biot, un groupe (C₁-C₆)alcoxy ou un groupe
- OSO₃⁻,
- R₃ représente l'enchaînement -T-Biot, un groupe (C₁-C₆)alcoxy,
- R₄ représente l'enchaînement -T-Biot, un groupe (C₁-C₆)alcoxy ou un groupe
- OSO₃⁻ ou bien R₄ constitue un pont -O-CH₂-, le groupe -CH₂- étant lié à l'atome de carbone porteur de la fonction carboxylique sur le même cycle ; étant entendu que l'un au moins des substituants R₁, R₂, R₃ ou R₄ représente un groupe -T-Biot,
- W représente un atome d'oxygène ou un groupe méthylène,
- T représente un des enchaînements choisis parmi: NH, 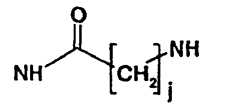
ou 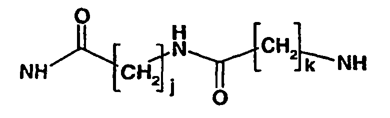 dans lesquels j et k, identiques ou différents, sont des entiers pouvant prendre toute valeur de 1 à 10 ;
- Biot représente le groupe : 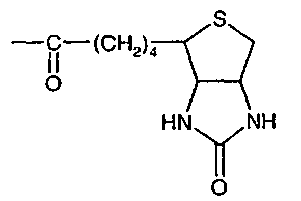 ainsi que leurs sels pharmaceutiquement acceptables.

3. Polysaccharides selon l'une des revendications 1 ou 2 de formule (I.1) : 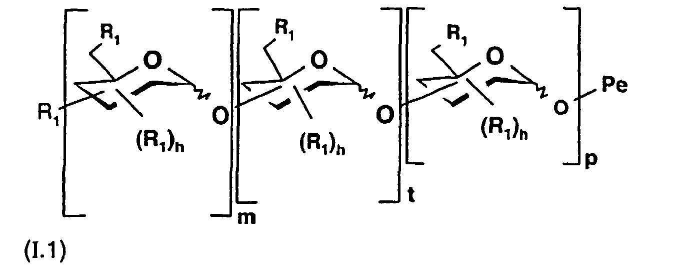 dans laquelle : 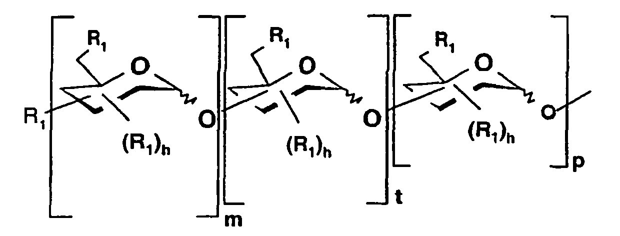 désigne une famille particulière de polysaccharides Po, liés par leur carbone anomère à Pe tel que défini pour (I), 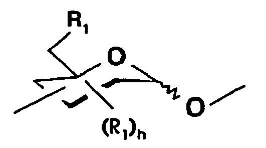 est tel que défini pour (I),
- les groupes R₁ sont tels que définis pour (I) et, pour un même monosaccharide, peuvent être identiques ou différents,
- le monosaccharide contenu dans [ ]ₘ est répété m fois, le monosaccharide contenu dans [ ]ₜ est répété t fois, le monosaccharide contenu dans [ ]ₚ est répété p fois,
- m est un entier variant de 1 à 5, t est un entier variant de 0 à 24 et p est un entier variant de 0 à 24 étant entendu que 1 ≤ m+t+p ≤ 25,
ainsi que leurs sels pharmaceutiquement acceptables.

4. Polysaccharides selon la revendication 3 **caractérisés en ce que** un seul des substituants R₁, R₂, R₃ ou R₄ représente l'enchaînement -T-Biot avec T et Biot étant tels que définis pour (I).

5. Hexadécasaccharides selon l'une quelconque des revendications 1 à 4 de formule (1.2) : 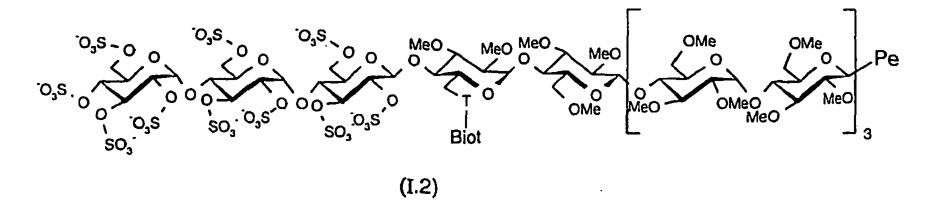 dans laquelle :
- T représente un des enchaînements choisis parmi : NH, 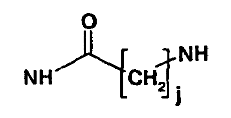
ou 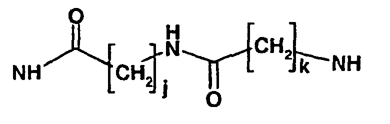 dans lesquels j et k, identiques ou différents, sont des entiers pouvant prendre toute valeur de 1 à 10 ;
- Biot représente le groupe : 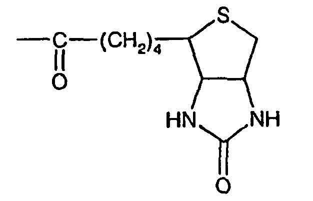
- Pe représente un pentasaccharide de structure : 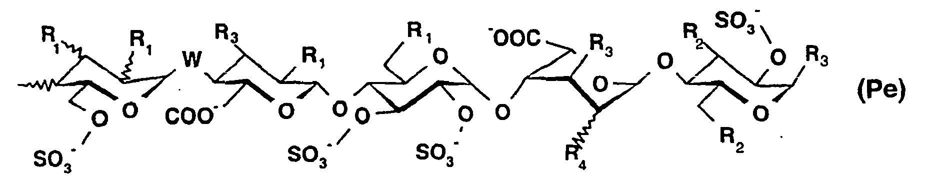
dans lequel :
- R₁ représente un groupe (C₁-C₆)alcoxy ou un groupe -OSO₃⁻,
- R₂ représente un groupe (C₁-C₆)alcoxy ou un groupe -OSO₃⁻,
- R₃ représente un groupe (C₁-C₆)alcoxy,
- R₄ représente un groupe (C₁-C₆)alcoxy ou un groupe -OSO₃⁻, ou bien R₄ constitue un pont -O-CH₂-, le groupe -CH₂ étant lié à l'atome de carbone porteur de la fonction carboxylique sur le même cycle,
- W représente un atome d'oxygène ou un groupe méthylène,
ainsi que leurs sels pharmaceutiquement acceptables.

6. Pentasaccharides selon l'une des revendications 1 ou 2 de formule (I.3): 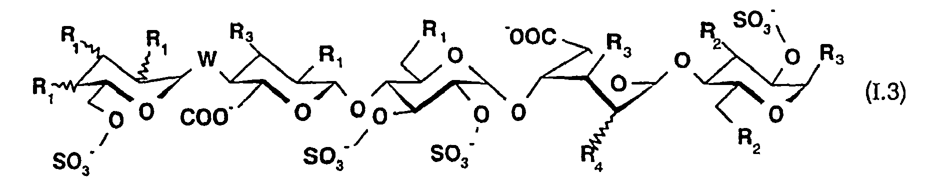 dans lesquels R₁, R₂, R₃, R₄ et W sont tels que définis pour (I), ainsi que leurs sels pharmaceutiquement acceptables.

7. Pentasaccharides selon la revendication 6 **caractérisé en ce que** un seul des substituants R₁, R₂, R₃ ou R₄ représente l'enchaînement -T-Biot avec T et Biot étant tels que définis pour (I).

8. Pentasaccharides selon l'une des revendications 6 ou 7 de formule (1.3) de formule (I.4) : 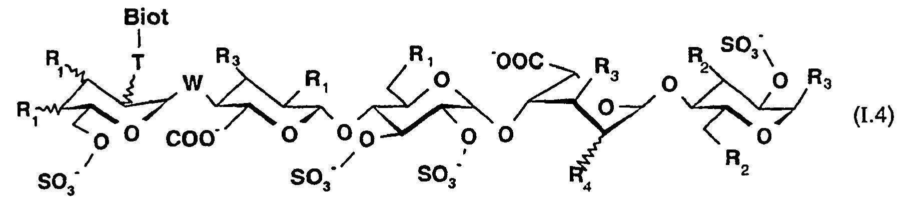 dans laquelle:
- T représente un des enchaînements choisis parmi : NH, 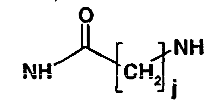
ou 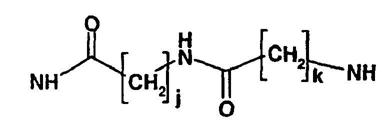 dans lesquels j et k, identiques ou différents, sont des entiers pouvant prendre toute valeur de 1 à 10 ;
- Biot représente le groupe : 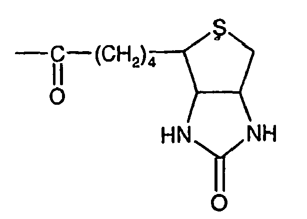
- R₁ représente un groupe (C₁-C₆)alcoxy ou un groupe -OSO₃⁻,
- R₂ représente un groupe (C₁-C₆)alcoxy ou un groupe -OSO₃⁻,
- R₃ représente un groupe (C₁-C₆)alcoxy,
- R₄ représente un groupe (C₁-C₆)alcoxy ou un groupe -OSO₃⁻, ou bien R₄ constitue un pont -O-CH₂-, le groupe -CH₂- étant lié à l'atome de carbone porteur de la fonction carboxylique sur le même cycle,
- W représente un atome d'oxygène ou un groupe méthylène,
ainsi que leurs sels pharmaceutiquement acceptables.

9. Polysaccharides selon l'une des revendications 1 ou 2 choisis parmi :
- Méthyl (2,3,4,6-tétra-*O*-sulfonato-α-D-glucopyranosyl)-(1-4)-(2,3,6-tri-*O*-sulfonato-α-D-glucopyranosyl)-(1→4)-(2,3,6-tri-*O*-sulfonato-β-D-glucopyranosyl)-(1→4)-(6-biotinamido-6-désoxy-2,3-di-*O*-méthyl-α-D-glucopyranosyl)-(1-4)-(2,3,6-tri-*O*-méthyl-β-D-glucopyranosyl)-(1→4)-[(2,3,6-tri-*O*-méthyl-α-D-glucopyranosyl)-(1->4)-*O*-(2,3,6-tri-*O-*méthy)-β-D-glucopyranosyl)-(1->4)]₃-(6-*O*-sulfonato-2,3-di-*O*-méthyl-α-D-glucopyrano-syl)-(1→4)-(acide 2,3-di- *O*-méthyl-β-D-glucopyranosyluronique)-(1→4)-(2,3, 6-tri-*O-*sulfonato-α-D-glucopyranosyl)-(1→4)-(acide 2,3-di-*O*-méthyl-α-L-idopyranosyl-uronique)-(1→4)-2,3,6-tri-*O*-sulfonato-α-D-glucopyranoside, sel de sodium,
- Méthyl (2,3,4,6-tétra-*O*-sulfonato-α-D-glucopyranosyl)-(1→4)-(2,3,6-tri-*O*-sulfonato-α-D-glucopyranosyl)-(1→4)-(2,3, 6-tri-*O*-sulfonato-β-D-glucopyranosyl)-(1→4)-(6-[6-(biotinamido)hexamido]-6-désoxy-2,3-di-*O*-méthyl-α-D-glucopyranosyl)-(1→4)-(2,3,6-tri-*O*-méthyl-β-D-glucopyranosyl)-(1→4)-[(2,3,6-tri-*O-*méthyl-α-D-glucopyranosyl)-(1→ 4)-*O*-(2,3,6-tri-*O*-méthyl-β-D-glucopyranosyl)-(1→4)]₃-(6-*O*-sulfonato-2,3-di-*O*-méthyl-α-D-glucopyranosyl)-(1→4)-(acide 2,3-di-*O*-méthyl-β-D-glucopyranosyluronique)-(1→ 4)-(2,3,6-tri-*O*-sulfonato-α-D-glucopyranosyl)-(1→4)-(acide 2,3-di-*O*-méthyl-α-L-ido-pyranosyluronique)-(1→4)-2,3,6-tri-*O*-sulfonato-α-D-glucopyranoside, sel de sodium,
- Méthyl (2,3,4,6-tétra-*O*-sulfonato-α-D-glucopyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(6-[6-(6-biotanamidohexamido) hexamido]-6-désoxy-2,3-di-*O*-méthyl-α-D-glucopyranosyl)-(1 →4)-(2,3,6-tri-*O*-méthyl-β-D-glucopyranosyl)-(1→4)-[(2,3,6-tri-*O*-méthyl-α-D-glucopyranosyl)-(1→4)-*O*-(2,3,6-tri-*O*-méthyl-β-D-glucopyranosyl)-(1→4)]₃-(6-*O-*sulfonato-2,3-di-*O*-méthy)-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O*-méthyl-α-L-idopyranosyturonique)-(1→4)-2,3,6-tri-*O*-sulfonato-α-D-glucopyranoside, sel de sodium,
- Méthyl (2-biotinamido-2-désoxy-3,4-di-O-méthyl-6-*O*-sulfonato-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O*-méthyl-α-L-idopyranosyluronique)-(1→4)-2,3,6-tri-*O*-sulfonato-α-D-glucopyranoside, sel de sodium,
- Méthyl (2-[6-(6-biotinamidohexamido) hexamido]-2-désoxy-3,4-di-*O*-méthyl-6-*O-*sulfonato-α-D-glucopyranosyl)-(1→4)-(acide 2,3-di-*O*-méthyl-β-D-glucopyranosyluronique)-(1→4)-(2,3,6-tri-*O*-sulfonato-α-D-glucopyranosyl)-(1→4)-(acide 2,3-di-*O-*méthy)-α-L-idopyranosyluronique)-(1→4)-2,3,6-tri-*O*-sulfonato-α-D-glucopyranoside, sel de sodium,
- Méthyl (2-[N-(6-biotinamido hexanoyl)]-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyra-nosyluronique)-(1→4)- 2,3,6-tri-O-sulfonato-α-D-glucopyranoside, sel de sodium.

10. Compositions pharmaceutiques contenant, en tant que principe actif, un polysaccharide selon l'une quelconque des revendications 1 à 9 éventuellement en association avec un ou plusieurs excipients inertes et appropriés.

11. Utilisation des compositions pharmaceutiques selon la revendication 10 pour la fabrication d'un médicament utile pour le traitement des pathologies consécutives à une modification de l'homéostasie du système de la coagulation apparaissant lors des troubles du système cardio-vasculaire et cérébro-vasculaire comme les troubles thromboemboliques associés à l'arthérosclérose et au diabète tels que l'angine instable, l'attaque cérébrale, la resténose après angioplastie, l'endartérectomie, la pose de prothèses endovasculaires, ou les troubles thromboemboliques associés à la rethrombose après thrombolyse, à l'infarctus, à la démence d'origine ischémique, aux maladies artérielles périphériques, à l'hémodialyse, aux fibrillations auriculaires, lors de l'utilisation de prothèses vasculaires de pontages aorto-coronariens, dans le traitement ou la prévention de pathologies thromboemboliques d'origine veineuse telles les embolies pulmonaires, pour prévenir ou pour traiter les complications thrombotiques observés à la suite d'opérations chirurgicales, de développements tumoraux ou de dérèglements de la coagulation, induits par des activateurs bactériens, viraux, ou enzymatiques.

12. Utilisation d'un polysaccharide selon l'une quelconque des revendications 1 à 9 pour recouvrir des prothèses.

13. Utilisation d'un polysaccharide selon l'une quelconque des revendications 1 à 9 comme adjuvants lors d'endartérectomie réalisée avec des ballonnets poreux.

14. Procédé mettant en oeuvre l'avidine ou la streptavidine **caractérisé en ce qu'**il permet de neutraliser les polysaccharides selon l'une quelconque des revendications 1 à 9.

15. Utilisation d'avidine ou de streptavidine pour la préparation de médicaments destinés à neutraliser les polysaccharides selon l'une quelconque des revendications 1 à 9.

16. Polysaccharide selon l'une des revendications 1 ou 2 de formule:

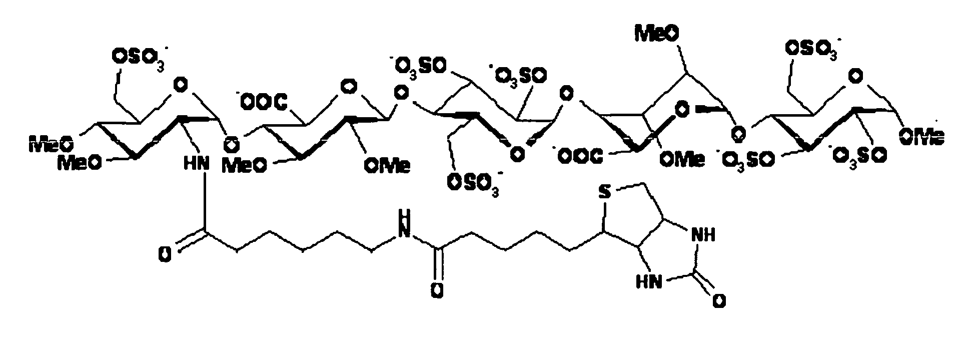

## Claims

1. Synthetic polysaccharides with antithrombotic activity and their pharmaceutically acceptable salts, **characterized in that** they exhibit at least one covalent bond with biotin or a biotin derivative.

2. Polysaccharides according to Claim 1 of formula: 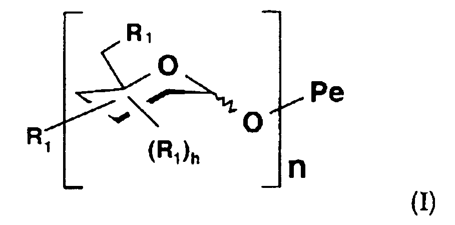 in which:
- the wavy line denotes a bond situated either below or above the plane of the pyranose ring, 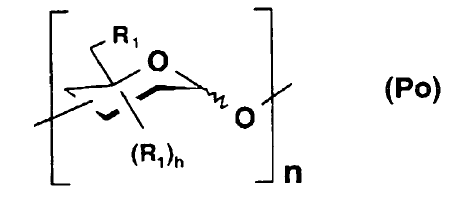 Po denotes a polysaccharide, comprising n identical or different monosaccharide units, bonded via its anomeric carbon to Pe, 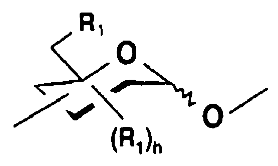 is a diagrammatic representation of a monosaccharide unit with a pyranose structure chosen from hexoses, pentoses and the corresponding deoxy sugars, this unit being bonded via its anomeric carbon to another monosaccharide unit and the hydroxyl groups of this unit being substituted by identical or different R₁ groups, R₁ being as defined below,
- Pe represents a pentasaccharide of structure: 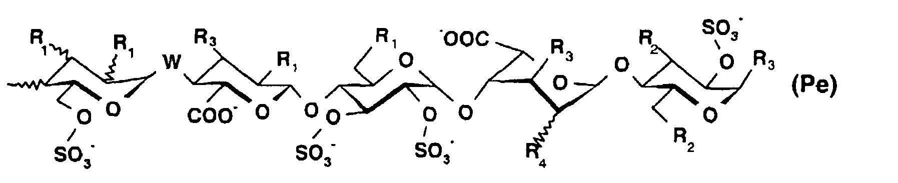
- h is equal to 1 or 2,
- n is an integer and can take any value from 0 to 25,
- R₁ represents the -T-Biot linkage, a (C₁-C₆)alkoxy group or an -OSO₃⁻ group,
- R₂ represents the -T-Biot linkage, a (C₁-C₆)alkoxy group or an -OSO₃⁻ group,
- R₃ represents the -T-Biot linkage or a (C₁-C₆) alkoxy group,
- R₄ represents the -T-Biot linkage, a (C₁-C₆)alkoxy group or an -OSO₃⁻ group, or else R₄ constitutes an -O-CH₂- bridge, the -CH₂- group being bonded to the carbon atom carrying the carboxyl functional group on the same ring; it being understood that at least one of the R₁, R₂, R₃ or R₄ substituents represents a -T-Biot group,
- W represents an oxygen atom or a methylene group,
- T represents one of the linkages chosen from: NH, 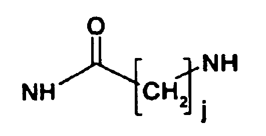 or 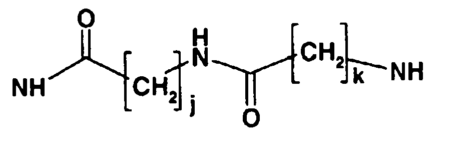
in which j and k, which are identical or different, are integers which can take any value from 1 to 10;
- Biot represents the group: 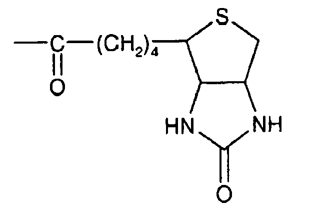 and their pharmaceutically acceptable salts.

3. Polysaccharides according to either of Claims 1 and 2 of formula (I.1): 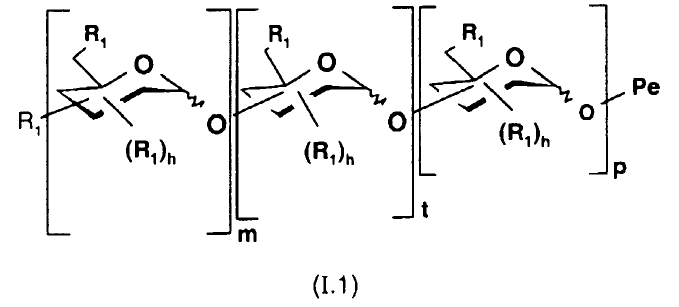 in which: 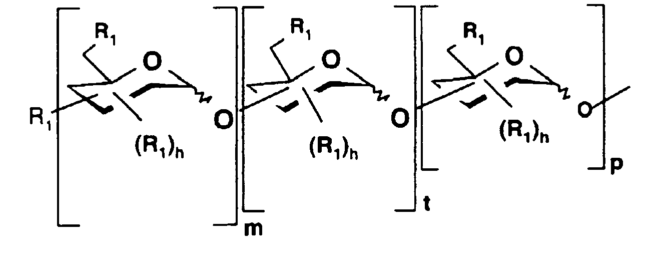 denotes a specific family of polysaccharides Po which are bonded via their anomeric carbon to Pe as defined for (I), 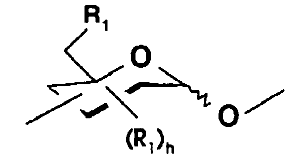 is as defined for (I),
- the R₁ groups are as defined for (I) and, for a same monosaccharide, can be identical or different,
- the monosaccharide present in []ₘ is repeated m times, the monosaccharide present in []ₜ is repeated t times and the monosaccharide present in []ₚ is repeated p times,
- m is an integer varying from 1 to 5, t is an integer varying from 0 to 24 and p is an integer varying from 0 to 24, it being understood that 1 ≤ m + t + p ≤ 25,
and their pharmaceutically acceptable salts.

4. Polysaccharides according to Claim 3, **characterized in that** only one of the R₁, R₂, R₃ or R₄ substituents represents the -T-Biot linkage with T and Biot being as defined for (I).

5. Hexadecasaccharides according to any one of Claims 1 to 4 of formula (1.2): 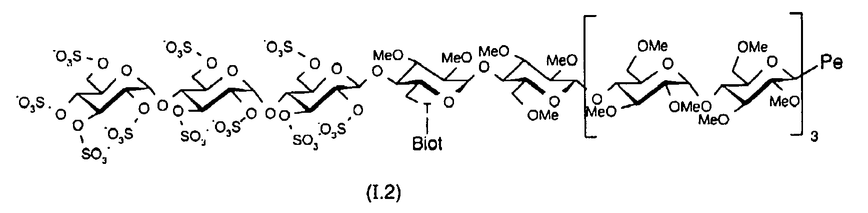 in which:
- T represents one of the linkages chosen from: NH, 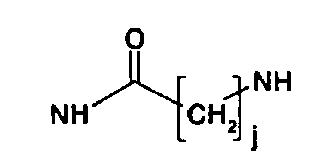 or 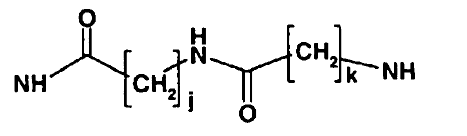
in which j and k, which are identical or different, are integers which can take any value from 1 to 10;
- Biot represents the group: 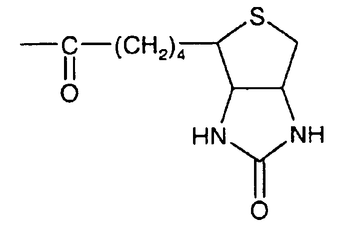
- Pe represents a pentasaccharide of structure: 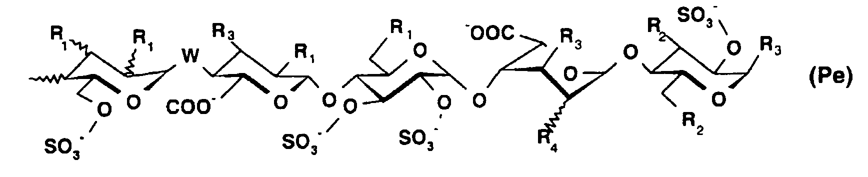
in which:
- R₁ represents a (C₁-C₆) alkoxy group or an
- OSO₃⁻ group,
- R₂ represents a (C₁-C₆) alkoxy group or an
- OSO₃⁻ group,
- R₃ represents a (C₁-C₆) alkoxy group,
- R₄ represents a (C₁-C₆) alkoxy group or an
- OSO₃⁻ group, or else R₄ constitutes an -O-CH₂-bridge, the -CH₂- group being bonded to the carbon atom carrying the carboxyl functional group on the same ring,
- W represents an oxygen atom or a methylene group,
and their pharmaceutically acceptable salts.

6. Pentasaccharides according to either of Claims 1 and 2 of formula (I.3) : 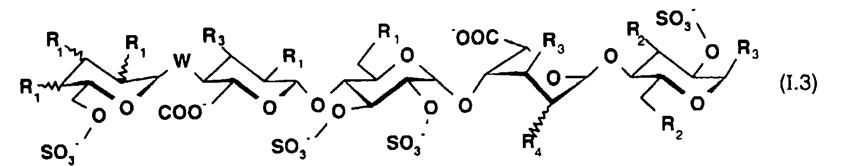 in which R₁, R₂, R₃, R₄ and W are as defined for (I), and their pharmaceutically acceptable salts.

7. Pentasaccharides according to Claim 6, **characterized in that** only one of the R₁, R₂, R₃ or R₄ substituents represents the -T-Biot linkage with T and Biot being as defined for (I).

8. Pentasaccharides according to either of Claims 6 and 7 of formula (1.4): 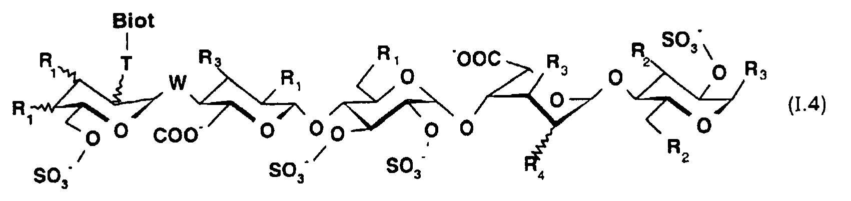 in which:
- T represents one of the linkages chosen from: NH, 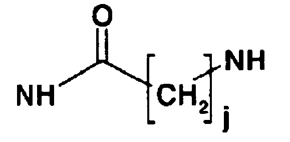 or 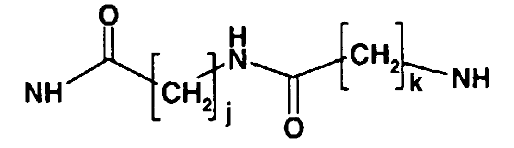
in which j and k, which are identical or different, are integers which can take any value from 1 to 10;
- Biot represents the group: 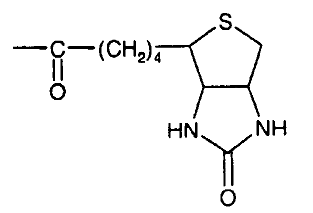
- R₁ represents a (C₁-C₆)alkoxy group or an -OSO₃⁻ group,
- R₂ represents a (C₁-C₆) alkoxy group or an -OSO₃⁻ group,
- R₃ represents a (C₁-C₆) alkoxy group,
- R₄ represents a (C₁-C₆)alkoxy group or an -OSO₃⁻ group, or else R₄ constitutes an -O-CH₂-bridge, the -CH₂- group being bonded to the carbon atom carrying the carboxyl functional group on the same ring,
- W represents an oxygen atom or a methylene group,
and their pharmaceutically acceptable salts.

9. Polysaccharides according to either of Claims 1 and 2 chosen from:
- Methyl (2,3,4,6-tetra-*O*-sulphonato-α-D-glucopyranosyl)-(1→4)-(2,3,6-tri-*O*-sulphonato-α-D-glucopyranosyl)-(1→4)-(2,3,6-tri-*O-*sulphonato-β-D-glucopyranosyl)-(1→4)-(6-biotinamido-6-deoxy-2,3-di-*O*-methyl-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O-*methyl-β-D-glucopyranosyl)-(1→4)]₃-(6-*O-*sulphonato-2,3-di-*O*-methyl-α-D-glucopyranosyl)-(1→4)-(2,3-di-*O*-methyl-β-D-glucopyranosyluronic acid)-(1→4)-(2,3,6-tri*O*-sulphonato-α-D-glucopyranosyl)-(1→4)-(2,3-di-*O*-methyl-α-L-idopyranosyluronic acid)-(1→4)-2,3,6-tri-*O*-sulphonato-α-D-glucopyranoside, sodium salt,
- Methyl (2,3,4,6-tetra-*O*-sulphonato-α-D-glucopyranosyl)-(1→4)-(2,3, 6-tri-*O*-sulphonato-α-D-glucopyranosyl)-(1→4)-(2,3,6-tri-*O-*sulphonato-β-D-glucopyranosyl)-(1→4)-(6-[6-(biotinamido)hexamido]-6-deoxy-2,3-di-*O-*methyl-α-D-glucopyranosyl)-(1→4)-(2,3,6-tri*O*-methyl-β-D-glucopyranosyl)-(1→4)-[(2,3,6-tri-*O*-methyl-α-D-glucopyranosyl)-(1→4)-*O-*(2,3,6-tri-*O*-methyl-β-D-glucopyranosyl)-(1→4)]₃-(6-*O*-sulphonato-2,3-di-*O*-methyl-α-D-glucopyranosyl)-(1→4)-(2,3-di-*O*-methyl-β-D-glucopyranosyluronic acid)-(1→4)-(2,3,6-tri*O*-sulphonato-α-D-glucopyranosyl)-(1→4)-(2,3-di-*O*-methyl-α-L-idopyranosyluronic acid)-(1→4)-2,3,6-tri-*O*-sulphonato-α-D-glucopyranoside, sodium salt,
- Methyl (2,3,4,6-tetra-*O*-sulphonato-α-D-glucopyranosyl)-(1→4)-(2,3,6-tri-*O*-sulphonato-α-D-glucopyranosyl)-(1→4)-(2,3,6-tri-*O-*sulphonato-β-D-glucopyranosyl)-(1→4)-(6-[6-(6-biotinamidohexamido)hexamido]-6-deoxy-2,3-di-*O*-methyl-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O*-methyl-β-D-glucopyranosyl)-(1→4)]₃-(6-*O*-sulphonato-2,3-di-*O*-methyl-α-D-glucopyranosyl)-(1→4)-(2,3-di-*O*-methyl-β-D-glucopyranosyluronic acid)-(1→4)-(2,3,6-tri-*O*-sulphonato-α-D-glucopyranosyl)-(1→4)-(2,3-di-*O*-methyl-α-L-idopyranosyluronic acid)-(1→4)-2,3,6-tri-*O-*sulphonato-α-D-glucopyranoside, sodium salt,
- Methyl (2-biotinamido-2-deoxy-3,4-di-*O-*methyl-6-*O*-sulphonato-α-D-glucopyranosyl)-(1→4)-(2,3-di-*O*-methyl-β-D-glucopyranosyluronic acid)-(1→4)-(2,3,6-tri*O*-sulphonato-α-D-glucopyranosyl)-(1→4)-(2,3-di-*O*-methyl-α-L-idopyranosyluronic acid)-(1→4)-2,3,6-tri-*O*-sulphonato-α-D-glucopyranoside, sodium salt,
- Methyl (2-[6-(6-biotinamidohexamido)hexamido]-2-deoxy-3,4-di-*O*-methyl-6-*O*-sulphonato-α-D-glucopyranosyl)-(1→4)-(2,3-di-*O*-methyl-β-D-glucopyranosyluronic acid)-(1→4)-(2,3, 6-tri-*O-*sulphonato-α-D-glucopyranosyl)-(1→4)-(2,3-di-*O*-methyl-α-L-idopyranosyluronic acid)-(1→4)-2,3,6-tri-*O*-sulphonato-α-D-glucopyranoside, sodium salt,
- Methyl (2-[N-(6-biotinamidohexanoyl]-2-deoxy-3,4-di-*O*-methyl-6-*O*-sulphonato-α-D-glucopyranosyl)-(1→4)-(2,3-di-*O*-methyl-β-D-glucopyranosyluronic acid)-(1→4)-(2,3,6-tri*O*-sulphonato-α-D-glucopyranosyl)-(1→4)-(2,3-di-*O*-methyl-α-L-idopyranosyluronic acid)-(1→4)-2,3,6-tri-*O*-sulphonato-α-D-glucopyranoside, sodium salt.

10. Pharmaceutical compositions comprising, as active principle, a polysaccharide according to any one of Claims 1 to 9, optionally in combination with one or more inert and appropriate excipients.

11. Use of the pharmaceutical compositions according to Claim 10 in the manufacture of a medicament of use in the treatment of pathologies resulting from a modification of the homoeostasis of the coagulation system appearing during disorders of the cardiovascular and cerebrovascular system, such as thromboembolic disorders associated with atherosclerosis and diabetes, for example unstable angina, apoplexy, postangioplasty restenosis, endarterectomy or the insertion of endovascular prostheses, or thromboembolic disorders associated with post-thrombolyses rethrombosis, with infarction, with dementia of ischaemic origin, with peripheral arterial diseases, with haemodialysis or with auricular fibrillations, during the use of vascular prostheses for aortocoronary bypasses, in the treatment or prevention of thromboembolic pathologies of venous origin, such as pulmonary embolisms, to prevent or treat the thrombotic complications observed following surgical operations, the growth of tumours or disturbances to coagulation induced by bacterial, viral or enzymatic activators.

12. Use of a polysaccharide according to any one of Claims 1 to 9, to cover prostheses.

13. Use of a polysaccharide according to any one of Claims 1 to 9 as adjuvants during endarterectomy carried out with porous balloons.

14. Process employing avidin or streptavidin, **characterized in that** it makes it possible to neutralize the polysaccharides according to any one of Claims 1 to 9.

15. Use of avidin or of streptavidin in the preparation of medicaments intended to neutralize the polysaccharides according to any one of Claims 1 to 9.

16. Polysaccharide according to either of Claims 1 and 2, of formula:

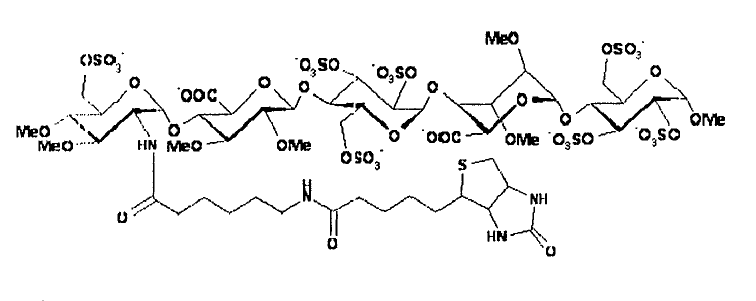

## Patentansprüche

1. Synthetische Polysaccharide mit antithrombotischer Wirkung und pharmazeutisch unbedenkliche Salze davon, **dadurch gekennzeichnet, daß** sie mindestens eine kovalente Bindung mit Biotin oder einem Biotinderivat aufweisen.

2. Polysaccharide nach Anspruch 1 der Formel: 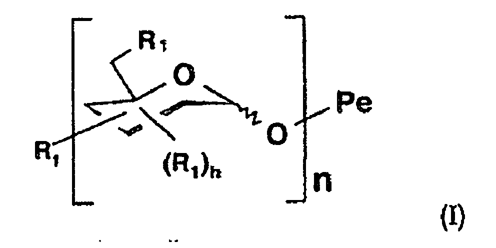 worin:
- die Wellenlinie für eine entweder oberhalb oder unterhalb der Ebene des Pyranoserings liegende Bindung steht, 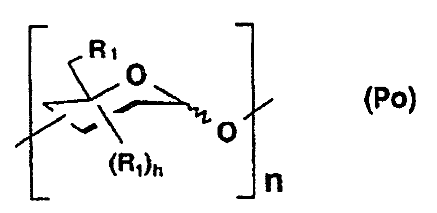 Po für ein Polysaccharid mit n gleichen oder verschiedenen Monosaccharideinheiten steht, das über seinen anomeren Kohlenstoff an Pe gebunden ist, 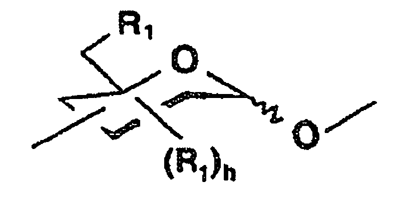 eine schematische Darstellung einer Monosaccharideinheit mit Pyranosestruktur, ausgewählt unter Hexosen, Pentosen und den entsprechenden Desoxyzuckern, ist, wobei diese Einheit über ihren anomeren Kohlenstoff an eine andere Monosaccharideinheit gebunden ist und die Hydroxygruppen dieser Einheit durch gleiche oder verschiedene Gruppen R₁ substituiert sein können, wobei R₁ die unten angegebene Bedeutung besitzt,
- Pe für ein Pentasaccharid der Struktur 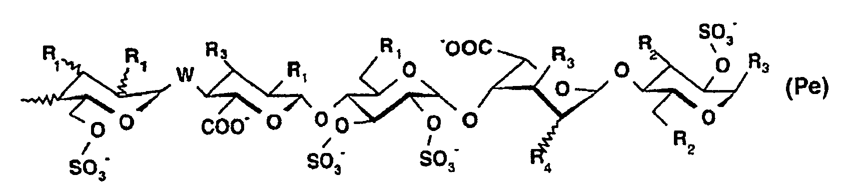 steht,
- h gleich 1 oder 2 ist,
- n für eine ganze Zahl steht und jeden Wert von 0 bis 25 annehmen kann,
- R₁ für die Kette -T-Biot, eine (C₁-C₆) -Alkoxygruppe oder eine OSO₃⁻-Gruppe steht,
- R₂ für die Kette -T-Biot, eine (C₁-C₆) -Alkoxygruppe oder eine OSO₃⁻-Gruppe steht,
- R₃ für die Kette -T-Biot oder eine (C₁-C₆) - Alkoxygruppe steht,
- R₄ für die Kette -T-Biot, eine (C₁-C₆) -Alkoxygruppe oder eine OSO₃⁻-Gruppe steht oder auch eine -O-CH₂-Brücke darstellt, wobei die -CH₂-Gruppe an das die Carboxylfunktion desselben Rings tragende Kohlenstoffatom gebunden ist; mit der Maßgabe, daß mindestens einer der Substituenten R₁, R₂, R₃ und R₄ für eine -T-Biot-Gruppe steht,
- W für ein Sauerstoffatom oder eine Methylengruppe steht,
- T für eine der unter
NH, 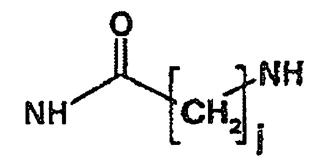 oder 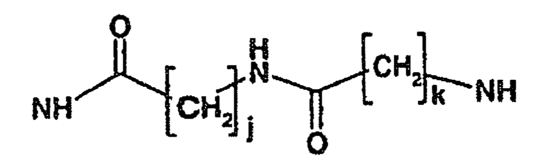
worin j und k gleich oder verschieden sind und für ganze Zahlen stehen, die jeden Wert von 1 bis 10 annehmen können, ausgewählten Ketten steht;
- Biot für die Gruppe: 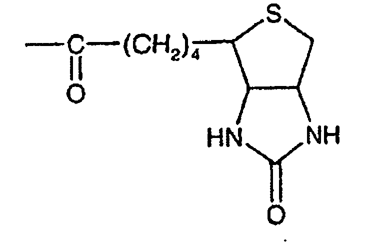 steht,
und pharmazeutisch unbedenkliche Salze davon.

3. Polysaccharide nach Anspruch 1 oder 2 der Formel (I.1) : 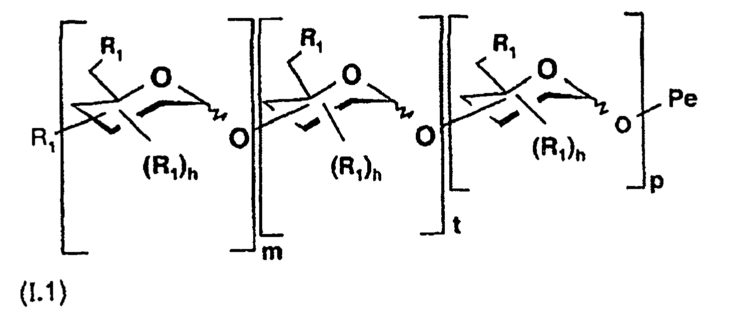 worin: 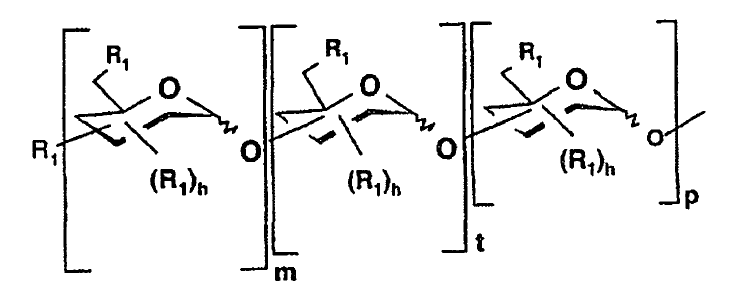 für eine spezielle Familie von Polysacchariden Po steht, die über ihren anomeren Kohlenstoff an Pe gebunden ist, wie für (I) definiert, 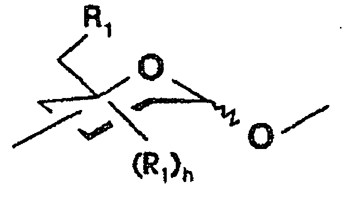 die unter (I) angegebene Bedeutung besitzt,
- die Gruppen R₁ die unter (I) angegebene Bedeutung besitzen und für ein und dasselbe Monosaccharid gleich oder verschieden sein können,
- das in []ₘ enthaltene Monosaccharid sich m-fach wiederholt, das in []ₜ enthaltene Monosaccharid sich t-fach wiederholt und das in []ₚ enthaltene Monosaccharid sich p-fach wiederholt,
- m für eine ganze Zahl von 1 bis 5 steht, t für eine ganze Zahl von 0 bis 24 steht und p für eine ganze Zahl von 0 bis 24 steht, mit der Maßgabe, daß 1 ≤ m + t + p ≤ 25,
und pharmazeutisch unbedenkliche Salze davon.

4. Polysaccharide nach Anspruch 3, **dadurch gekennzeichnet, daß** nur einer der Substituenten R₁, R₂, R₃ und R₄ für die Kette -T-Biot steht, wobei T und Biot die unter (I) angegebene Bedeutung besitzen.

5. Hexadecasaccharide nach einem der Ansprüche 1 bis 4 der Formel (1.2). 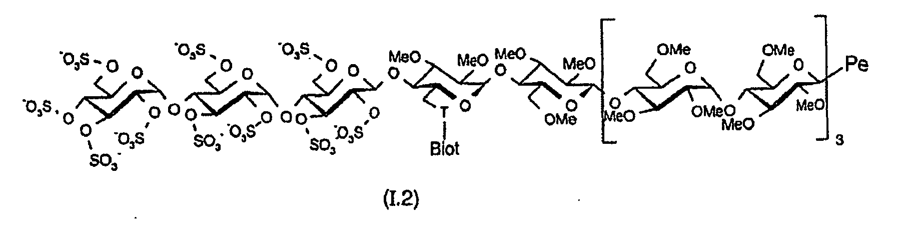 worin:
- T für eine der unter NH, 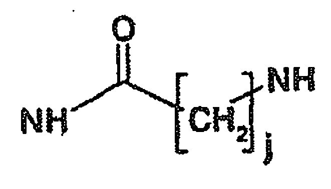 oder 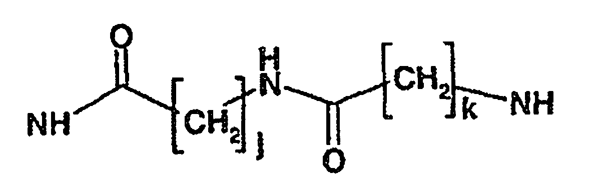
worin j und k gleich oder verschieden sind und für ganze Zahlen stehen, die jeden Wert von 1 bis 10 annehmen können, ausgewählten Ketten steht;
- Biot für die Gruppe: 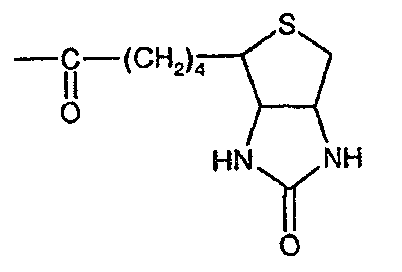 steht,
- Pe für ein Pentasaccharid der Struktur 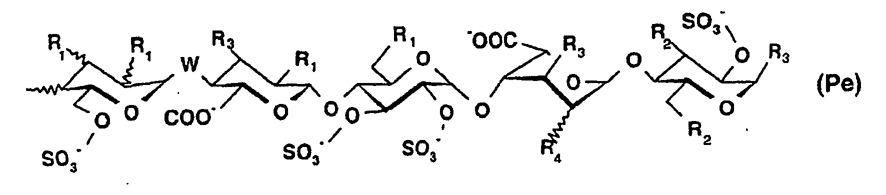 steht, worin
- R₁ für eine (C₁-C₆)-Alkoxygruppe oder eine OSO₃⁻-Gruppe steht,
- R₂ für eine (C₁-C₆) -Alkoxygruppe oder eine OSO₃⁻-Gruppe steht,
- R₃ für eine (C₁-C₆)-Alkoxygruppe steht,
- R₄ für eine (C₁-C₆)-Alkoxygruppe oder eine OSO₃⁻-Gruppe steht oder auch eine -O-CH₂-Brücke darstellt, wobei die -CH₂-Gruppe an das die Carboxylfunktion desselben Rings tragende Kohlenstoffatom gebunden ist;
- W für ein Sauerstoffatom oder eine Methylengruppe steht,
und pharmazeutisch unbedenkliche Salze davon.

6. Pentasaccharide nach Anspruch 1 oder 2 der Formel (I.3): 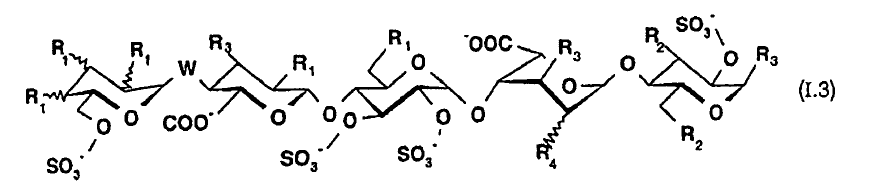 worin R₁, R₂, R₃ und R₄ die unter (I) angegebene Bedeutung besitzen, und pharmazeutisch unbedenkliche Salze davon.

7. Pentasaccharide nach Anspruch 6, **dadurch gekennzeichnet, daß** nur einer der Substituenten R₁, R₂, R₃ und R₄ für die Kette -T-Biot steht, wobei T und Biot die unter (I) angegebene Bedeutung besitzen.

8. Pentasaccharide nach Anspruch 6 oder 7 der Formel (1.3) der Formel (1.4): 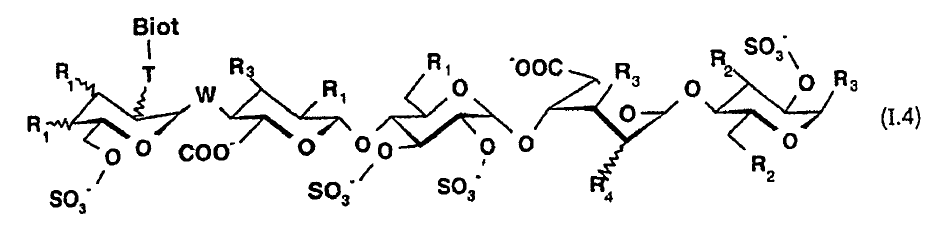 worin:
- T für eine der unter
NH, 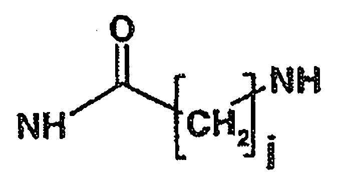 oder 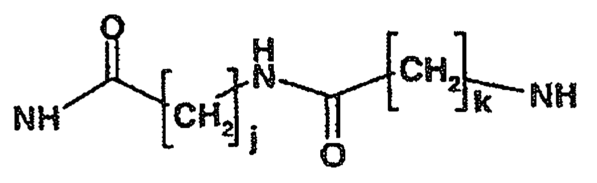
worin j und k gleich oder verschieden sind und für ganze Zahlen stehen, die jeden Wert von 1 bis 10 annehmen können, ausgewählten Ketten steht;
- Biot für die Gruppe: 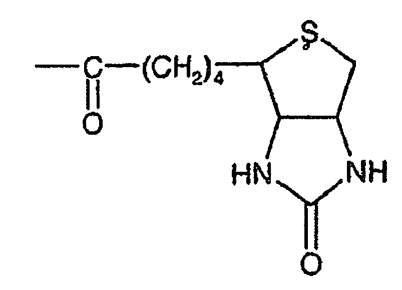 steht,
- R₁ für eine (C₁-C₆) -Alkoxygruppe oder eine OSO₃⁻-Gruppe steht,
- R₂ für eine (C₁-C₆) -Alkoxygruppe oder eine OSO₃⁻-Gruppe steht,
- R₃ für eine (C₁-C₆) -Alkoxygruppe steht,
- R₄ für eine (C₁-C₆) -Alkoxygruppe oder eine OSO₃⁻-Gruppe steht oder auch eine -O-CH₂-Brücke darstellt, wobei die -CH₂-Gruppe an das die Carboxylfunktion desselben Rings tragende Kohlenstoffatom gebunden ist;
- W für ein Sauerstoffatom oder eine Methylengruppe steht,
und pharmazeutisch unbedenkliche Salze davon.

9. Polysaccharide nach Anspruch 1 oder 2, ausgewählt unter:
- Methyl-(2,3,4,6-tetra-*O*-sulfonato-α-D-glucopyranosyl)-(1→4)-(2,3,6-tri-O-sulfonato-α-D-glucopyranosyl)- (1→4) - (2, 3, 6-tri-O-sulfonato-β-D-glucopyranosyl)-(1→4)-(6-biotinamido-6-desoxy-2,3-di-*O*-methyl-α-D-glucopyranosyl) - (1→4) - (2, 3, 6-tri-*O*-methyl-β-D-glucopyranosyl)-(1→4)-[(2,3,6-tri-*O*-methyl-α-D-gluco-pyranosyl) - (1→4)-*O*-(2,3,6-tri*-O*-methyl-β-D-glucopyranosyl)-(1→4)]₃-(6-*O*-sulfonato-2,3-di-*O*-methyl-α-D-glucopyranosyl)-(1→4)-(2,3-di-*O-*methyl-β-D-glucopyranosyluronsäure)-(1→4)-(2,3,6-tri-*O*-sulfonato-α-D-glucopyranosyl)-(1→4)-(2,3-di-*O*-methyl-α-L-idopyranosyl-uronsäure)-(1→4)-2,3,6-tri-*O*-sulfonato-α-D-glucopyranosid-Natriumsalz,
- Methyl-(2,3,4,6-tetra-*O*-sulfonato-α-D-gluco-pyranosyl)-(1→4)-(2,3,6-tri-*O*-sulfonato-α-D-glucopyranosyl)-(1→4)-(2,3,6-tri-*O*-sulfonato-β-D-glucopyranosyl)-(1→4)-(6-[6-(biotinamido)-hexamido]-6-desoxy-2,3-di-*O*-methyl-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-*O*-(2,3,6-tri-*O*-methyl-β-D-glucopyranosyl)-(1→4)]3-(6-*O*-sulfonato-2,3-di-*O*-methyl-α-D-glucopyranosyl)-(1→4)-(2,3-di-*O*-methyl-β-D-glucopyranosyluronsäure)-(1→4)-(2,3,6-tri-*O*-sulfonato-α-D-glucopyranosyl)-(1→4)-(2,3-di-*O*-methyl-α-L-idopyranosyluronsäure)-(1→4)-2,3,6-tri-*O*-sulfonato-α-D-glucopyranosid-Natriumsalz,
- Methyl-(2,3,4,6-tetra-*O*-sulfonato-α-D-glucopyranosyl)-(1→4)-(2,3,6-tri-O-sulfonato-α-D-glucopyranosyl)-(1→4)-(2,3,6-tri-*O*-sulfonato-β-D-glucopyranosyl)-(1→4)-(6-[6-(6-biotinamidohexamido)hexamido]-6-desoxy-2,3-di-*O*-methyl-α-D-glucopyranosyl)-(1→4)-(2,3,6-tri-*O*-methyl-β-D-glucopyranosyl)-(1→4)-[(2,3,6-tri-*O*-methyl-α-D-glucopyranosyl)-(1→4)-O-(2,3,6-tri-*O-*methyl-β-D-glucopyranosyl)-(1→4)]₃-(6-*O-*sulfonato-2,3-di-*O*-methyl-α-D-glucopyranosyl)-(1→4)-(2,3-di-*O*-methyl-β-D-glucopyranosyl-uronsäure)-(1→4)-(2,3,6-tri-*O*-sulfonato-α-D-glucopyranosyl)-(1→4)-(2,3-di-*O*-methyl-α-L-idopyranosyluronsäure)-(1→4)-2,3,6-tri-*O-*sulfonato-α-D-glucopyranosid-Natriumsalz,
- Methyl-(2-biotinamido-2-desoxy-3,4-di-*O*-methyl-6-*O*-sulfonato-α-D-glucopyranosyl)-(1→4)-(2,3-di-*O*-methyl-β-D-glucopyranosyluronsäure)-(1→4)-(2,3,6-tri-*O*-sulfonato-α-D-glucopyranosyl)-(1→4)-(2,3-di-*O*-methyl-α-L-idopyranosyluronsäure)-(1→4)-2,3,6-tri-*O-*sulfonato-α-D-glucopyranosid-Natriumsalz,
- Methyl-(2-[6-(6-biotinamidohexamido)hexamido]-2-desoxy-3,4-di-*O*-methyl-6-*O*-sulfonato-α-D-glucopyranosyl)-(1→4)-(2,3-di-*O*-methyl-β-D-glucopyranosyluronsäure)-(1→4)-(2,3,6-tri-*O-*sulfonato-α-D-glucopyranosyl)-(1→4)-(2,3-di-*O-*methyl-α-L-idopyranosyluronsäure)-(1→4)-2,3,6-tri-O-sulfonato-α-D-glucopyranosid-Natriumsalz,
- Methyl-(2-[N-(6-biotinamidohexanoyl)]-2-desoxy-3,4-di-*O*-methyl-6-*O*-sulfonato-α-D-glucopyranosyl)-(1→4)-(2,3-di-O-methyl-β-D-glucopyranosyluronsäure)-(1→4)-(2,3,6-tri-O-sulfonato-α-D-glucopyranosyl)-(1→4)-(2,3-di-O-methyl-α-L-idopyranosyluronsäure)-(1→4)-2,3,6-tri-O-sulfonato-α-D-glucopyranosid-Natriumsalz.

10. Pharmazeutische Zusammensetzungen, enthaltend als Wirkstoff ein Polysaccharid nach einem der Ansprüche 1 bis 9, gegebenenfalls in Kombination mit einem oder mehreren inerten und geeigneten Trägerstoffen.

11. Verwendung der pharmazeutischen Zusammensetzungen nach Anspruch 10 zur Herstellung eines Arzneimittels zur Behandlung von Folgepathologien einer Modifizierung der Homöostase des Koagulationssystems, die bei Störungen des kardiovaskulären oder zerebrovaskulären Systems wie mit Arteriosklerose und Diabetes assoziierten thromboembolischen Störungen, wie instabiler Angina, Hirnschlag, Restenose nach Angioplastie, Endarteriektomie oder dem Einsetzen von endovaskulären Prothesen, oder mit Rethrombose nach Thrombolyse, Infarkt, Demenz ischämischen Ursprungs, peripheren arteriellen Erkrankungen, Hämodialyse oder Vorhofflimmern assoziierten thromboembolischen Störungen oder bei der Verwendung von Gefäßprothesen bei aortokoronaren Bypässen auftreten, bei der Behandlung oder Prävention von thromboembolischen Erkrankungen venösen Ursprungs wie Lungenembolien, zur Prävention oder Behandlung von thrombotischen Komplikationen nach chirurgischen Operationen, Tumorwachstum oder durch bakterielle, virale oder enzymatische Aktivatoren induzierten Koagulationsstörungen.

12. Verwendung eines Polysaccharids nach einem der Ansprüche 1 bis 9 zur Beschichtung von Prothesen.

13. Verwendung eines Polysaccharids nach einem der Ansprüche 1 bis 9 als Adjuvantien bei der Endarteriektomie mit porösen Ballons.

14. Verfahren unter Verwendung von Avidin oder Streptavidin, **dadurch gekennzeichnet, daß** es die Neutralisation der Polysaccharide nach einem der Ansprüche 1 bis 9 erlaubt.

15. Verwendung von Avidin oder Streptavidin zur Herstellung von Arzneimitteln zur Neutralisation der Polysaccharide nach einem der Ansprüche 1 bis 9.

16. Polysaccharide nach Anspruch 1 oder 2 der Formel:
